(19) Europäisches Patentamt
European Patent Office
Office européen des brevets

(11) EP 1 329 516 A1

(12) **EUROPEAN PATENT APPLICATION**
published in accordance with Art. 158(3) EPC

(43) Date of publication:
**23.07.2003 Bulletin 2003/30**

(21) Application number: **01974664.3**

(22) Date of filing: **03.10.2001**

(51) Int Cl.[7]: **C12Q 1/68**, C12N 15/09, C12M 1/00, G01N 33/15, G01N 33/50, G01N 33/53, G01N 33/566, A61K 31/351, A61K 45/00

(86) International application number:
**PCT/JP01/08698**

(87) International publication number:
**WO 02/029099 (11.04.2002 Gazette 2002/15)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **03.10.2000 JP 2000303644**
**18.09.2001 JP 2001283393**

(71) Applicant: **TAKARA BIO INC.**
**Otsu-shi, Shiga 520-2193 (JP)**

(72) Inventors:
- **ENOKI, Tatsuji**
**Otsu-shi, Shiga 520-0865 (JP)**
- **YAMASHITA, Shusaku**
**Shiga 520-2263 (JP)**
- **ROKUSHIMA, Masatomo**
**Otsu-shi, Shiga 520-2133 (JP)**
- **MINENO, Junichi**
**Uji-shi, Kyoto 611-0002 (JP)**
- **SAGAWA, Hiroaki**
**Kusatsu-shi, Shiga 525-0025 (JP)**
- **KATO, Ikunoshin**
**Uji-shi, Kyoto 611-0028 (JP)**

(74) Representative: **VOSSIUS & PARTNER**
**Siebertstrasse 4**
**81675 München (DE)**

(54) **METHOD OF SCREENING PHYSIOLOGICALLY ACTIVE SUBSTANCE**

(57) The present invention provides a method of screening a biologically active substance or a candidate substance for a biologically active substance, possessing an activity for controlling gene expression to the same extent to that of DGE; a gene controlling agent which is effective for maintaining homeostasis in a living body, for instance, purposed for mitigation or prevention of stress; and a method of controlling a gene.

EP 1 329 516 A1

## Description

### TECHNICAL FIELD

The present invention relates to a method of screening a biologically active substance, a gene controlling agent and a method of controlling a gene.

### BACKGROUND ART

An organism is subjected to various stresses caused by external factors such as change in living environment and internal factors such as viral and bacterial infections. The stress itself is a factor which hinders a normal life of an organism, and when the stress cannot be well avoided or when excessive reaction rather takes place, a more severe disorder, for instance, a disease is caused.

In a state where stress is loaded or in a state where the stage is progressed to a disease, various functions and elements in a living body are involved in a compound manner, and there basically exists a change in the gene expression state. Therefore, the stress can be supposedly avoided smoothly by regulation on the gene level. However, the details of the regulation of gene expression in the state under stress, and in the process where the stress is avoided are not clarified. Therefore, a means of avoiding stress by the regulation of gene level as mentioned above has not yet been established.

On the other hand, L-glycero-1,5-epoxy-1αβ,6-dihydroxy-cis-hex-3-en-2-one (hereinafter referred to as DGE), which can be obtained from agarose, has been known as a compound that possesses various kinds of biological activities, and is useful for maintaining homeostasis in a living body (WO 99/64424). However, the action mechanism on the gene level of the compound has not been clarified.

An object of the present invention is to provide a method of screening a biologically active substance or a candidate substance for a biologically active substance, possessing an activity for controlling gene expression to the same extent to that of DGE; a gene controlling agent which is effective for maintaining homeostasis in a living body, for instance, purposed for mitigation or prevention of stress; and a method of controlling a gene.

### DISCLOSURE OF INVENTION

A first invention of the present invention relates to a method of screening a biologically active substance, which comprises the following steps:

(1) loading to a living body either 1) a test substance or 2) a compound of the following (i):

(i) at least one compound selected from the group consisting of a compound represented by the following formula (I):

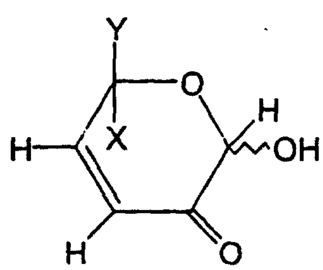

(I)

wherein X and Y are H or $CH_2OH$, with proviso that when X is $CH_2OH$, Y is H, and when X is H, Y is $CH_2OH$, a derivative thereof and a salt thereof; and/or
a compound of the following (ii):
(ii) at least one compound selected from the group consisting of 3,6-anhydrogalactose represented by the following formula (II):

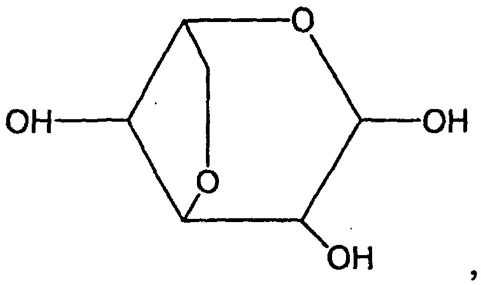 (II)

,

an aldehyde thereof, a hydrate thereof, and a 2-O-methylated product and a 2-O-sulfated product of the foregoing compounds; and/or at least one compound selected from the group consisting of soluble saccharide compounds having the above compound at reducing end;

(2) determining an expression level of a gene in the living body of (1), and comparing resulting values between a case where 1) is loaded and a case where 2) is loaded; and
(3) selecting a test substance which causes a change in an expression level of at least one gene to substantially the same extent to a change where 2) is loaded.

A second invention of the present invention relates to a method of screening a biologically active substance, which comprises the following steps:

(1) loading a test substance to a living body;
(2) determining an expression level of a gene in the living body of the above (1) and an expression level of a gene in the living body without the loading, and comparing resulting values therebetween; and
(3) selecting a test substance which decreases an expression level of at least one gene selected from the following Group A, Group C, Group E and Group F, and/or increases an expression level of at least one gene selected from the following Group B, Group D and Group G:

Group A:

Bone morphogenetic protein-6 [BMP-6 (GenBank Accession No. NM_001718)] gene,
Chemokine receptor-1 [CCR-1 (GenBank Accession No. NM_001295)] gene,
Hepatocyte growth factor [HGF (GenBank Accession No. X16323)] gene,
Intercellular adhesion molecule-1 [ICAM-1 (GenBank Accession No. NM_000201)] gene,
Interleukin-1β [IL-1β (GenBank Accession No. X02532)] gene,
Interleukin-16 [IL-16 (GenBank Accession No. M90391)] gene,
Leukemia inhibitory factor [LIF (GenBank Accession No. NM_002309)] gene,
Osteopontin [Osteopontin (GenBank Accession No. NM_000582)] gene,
Tumor necrosis factor-α [TNF-α (GenBank Accession No. X02910)] gene,
Neuropilin-1 [Neuropilin-1 (GenBank Accession No. NM_003873)] gene, signal transducer and activator of transcription 6 [STAT6 (GenBank Accession No. AF067575)] gene, and
Gene containing Homo Sapiens cDNA: FLJ22298 fis, clone HRC04637 (GenBank Accession No. AK025951),

Group B:

Heme oxygenase-1 [HO-1 (GenBank Accession No. NM_002133)] gene,
Thioredoxin reductase-1 (GenBank Accession No. AF106697) gene, putative translation initiation factor [SUI1 (GenBank Accession No. AF083441)] gene, and
Homo sapiens hypothetical protein [HSPC014 (GenBank Accession No. NM_015932)] gene;

Group C:

N-myc downstream regulated gene 1 [NDRG1 (GenBank Accession No. NM_006096)] gene,

adenosine deaminase, RNA specific [ADAR (GenBank Accession No. NM_015841)] gene,
splicing factor 3b, subunit2 [SF3B2 (GenBank Accession No. NM_006842)] gene,
interleukin enhancer binding factor 3 [ILF3 (GenBank Accession No. NM_004516)] gene,
valyl-tRNA synthetase 2 [VARS2 (GenBank Accession No. NM_006295)] gene, and
Gene containing the nucleotide sequence shown in SEQ ID NO: 12 of Sequence Listing;

Group D:

eukaryotic translation elongation factor 1 alpha 1 [EEF1A1 (GenBank Accession No. NM_001402)] gene,
ribosomal protein L17 [RPL17 (GenBank Accession No. NM_000985)] gene,
ribosomal protein L24 [RPL24 (GenBank Accession No. NM_000986)] gene,
splicing factor, arginine/serine-rich 2 [SFRS2 (GenBank Accession No. NM_003016)] gene,
tumor protein, translationally-controlled 1 [TPT1 (GenBank Accession No. NM_003295)] gene,
ubiquinol-cytochrome c reductase binding protein [UQCRB (GenBank Accession No. NM_006294)] gene,
ribosomal protein S15a [RPS15A (GenBank Accession No. NM_001019)] gene, and
ribosomal protein L27 [RPL27 (GenBank Accession No. NM_000988)] gene;

Group E:

Interleukin-2 receptor α [IL2RA (GenBank Accession No. X01057)] gene,
Interleukin-6 [IL6 (GenBank Accession No. X04430)] gene,
CD166 (GenBank Accession No. Y10183) gene,
Interferon regulatory factor-1 [IRF1 (GenBank Accession No. X14454)] gene,
Interleukin-15 receptor α [IL15RA (GenBank Accession No. U31628)] gene,
Nuclear factor kappa-b p105 Subunit [NFKB1 (GenBank Accession No. M58603)] gene,
Caspase-1 [CASP1 (GenBank Accession No. M87507)] gene,
Interleukin-1β [IL1B (GenBank Accession No. M15330)] gene,
Tumor necrosis factor-α [TNF-α (GenBank Accession No. X02910)] gene,
Gro1 oncogene [GRO1 (GenBank Accession No. X54489)] gene,
Interleukin-1α [IL1A (GenBank Accession No. M28983)] gene,
Inhibin βA [INHBA (GenBank Accession No. J03634)] gene,
Interleukin-7 receptor [IL7R (GenBank Accession No. M29696)] gene,
Precursor B cell colony enhancing factor [PBEF (GenBank Accession No. U02020)] gene,
Liver and activation regulated chemokine [LARC (GenBank Accession No. U64197)] gene, and
Interleukin-8 [IL8 (GenBank Accession No. M26383)] gene;

Group F:

Colony-stimulating factor-1 receptor [CSF1R (GenBank Accession No. X03663)] gene,
Chemokine (C-X-C motif) receptor-4 [CXCR4 (GenBank Accession No. AF147204)] gene, and
Endoglin [ENG (GenBank Accession No. NM_000118)] gene; and

Group G:

Heme oxygenase-1 [HMOX1 (GenBank Accession No. Z82244)] gene.

A third invention of the present invention relates to a method of screening a biologically active substance, which comprises the following steps:

(1) loading stress to a living body, and loading a test substance with stress to a living body, respectively;
(2) determining each of expression levels of a gene in the living body of the above (1) and in a normal living body without any of the loading; and
(3) comparing the expression levels of a gene obtained in the above (2) between the living body of the above (1) and the normal living body, and selecting a test substance showing at least one gene expression pattern selected from the gene expression patterns of the followings (a) to (g):

(a) an expression level of at least one gene selected from Group A as defined above increases when stress is loaded to a living body, and decreases when a test substance and stress are loaded to a living body, as

compared to that of the normal living body;

(b) an expression level of at least one gene selected from Group B as defined above increases when stress is loaded to a living body, and further increases when a test substance and stress are loaded to a living body, as compared to that of the normal living body;

(c) an expression level of at least one gene selected from Group C as defined above decreases when stress is loaded to a living body, and further decreases when a test substance and stress are loaded to a living body, as compared to that of the normal living body;

(d) an expression level of at least one gene selected from Group D as defined above decreases when stress is loaded to a living body, and increases when a test substance and stress are loaded to a living body, as compared to that of the normal living body;

(e) an expression level of at least one gene selected from Group E as defined above increases when stress is loaded to a living body, and decreases when a test substance and stress are loaded to a living body, as compared to that of the normal living body;

(f) an expression level of at least one gene selected from Group F as defined above is the same extent to that of the normal living body when stress is loaded to a living body, and decreases when a test substance and stress are loaded to a living body, as compared to that of the normal living body; and

(g) an expression level of at least one gene selected from Group G as defined above is the same extent to that of the normal living body when stress is loaded to a living body, and increases when a test substance and stress are loaded to a living body, as compared to that of the normal living body.

In the third invention of the present invention, when the stress loaded to a living body is a treatment with phorbol myristate acetate, the gene expression pattern is exemplified by the above (a) to (d), and when the stress is a treatment with lipopolysaccharide, the gene expression pattern is exemplified by the above (e) to (g).

In the first to third inventions of the present invention, as the living body, there is exemplified an organism individual or a cultured cell. As the gene expression level, there is exemplified an expression level of a gene determined by an amount of mRNA transcribed from the gene. It is especially preferable that the expression level is determined by hybridization method using a DNA array. Also, the present invention encompasses substances which can be obtained by the first to third inventions of the present invention.

A fourth invention of the present invention relates to a DNA array usable for the screening method of the first to third inventions of the present invention, characterized in that at least two genes selected from the genes of Groups A to G as defined above or fragments thereof are immobilized on a support at a given position.

A fifth invention of the present invention relates to a gene controlling agent characterized in that the gene controlling agent comprises a compound possessing an action of decreasing an expression level of at least one gene selected from Group A, Group C, Group E and Group F as defined above, and/or increasing an expression level of at least one gene selected from Group B, Group D and Group G as defined above.

A sixth invention of the present invention relates to a gene controlling agent characterized in that the gene controlling agent comprises a compound possessing an action of decreasing an expression level of at least one gene selected from Group A, Group C, Group E and Group F as defined above, and/or increasing an expression level of at least one gene selected from Group B, Group D and Group G as defined above, in a living body to which stress is loaded.

A seventh invention of the present invention relates to a gene controlling agent characterized in that the gene controlling agent comprises a compound possessing an action of decreasing an expression level of at least one gene selected from Group A and Group E as defined above in a living body to which stress is loaded, wherein the expression level of at least one gene is increased by the stress.

An eighth invention of the present invention relates to a gene controlling agent characterized in that the gene controlling agent comprises a compound possessing an action of increasing an expression level of at least one gene selected from Group B and Group G as defined above in a living body to which stress is loaded, wherein the expression level of at least one gene is increased by the stress.

A ninth invention of the present invention relates to a gene controlling agent characterized in that the gene controlling agent comprises a compound possessing an action of decreasing an expression level of at least one gene selected from Group C and Group F as defined above in a living body to which stress is loaded, wherein the expression level of at least one gene is decreased by the stress.

A tenth invention of the present invention relates to a gene controlling agent characterized in that the gene controlling agent comprises a compound possessing an action of increasing an expression level of at least one gene selected from Group D as defined above in a living body to which stress is loaded, wherein the expression level of at least one gene is decreased by the stress.

In the fifth to tenth inventions of the present invention, there is exemplified a gene controlling agent in which each compound contained is a substance obtained by the screening method of any one of the first to third inventions of the present invention. Also, as the gene controlling agent of the fifth to tenth inventions of the present invention,

there is exemplified a gene control agent which is purposed for mitigating or preventing stress.

An eleventh invention of the present invention relates to a method of controlling a gene characterized by administering to a living body either at least one compound selected from the group consisting of compounds of (i) and (ii) as defined above and a compound possessing an activity for controlling gene expression to substantially the same extent to that of the compounds for at least one gene, or a composition comprising the compound, thereby decreasing an expression level of at least one gene selected from Group A, Group C, Group E and Group F as defined above, and/or increasing an expression level of at least one gene selected from Group B, Group D and Group G, wherein Groups A to G are as defined above.

A twelfth invention of the present invention relates to a method of controlling a gene characterized by administering to a living body to which stress is loaded either at least one compound selected from the group consisting of compounds of (i) and (ii) as defined above and a compound possessing an activity for controlling gene expression to substantially the same extent to that of the compounds for at least one gene, or a composition comprising the compound, thereby decreasing an expression level of at least one gene selected from Group A, Group C, Group E and Group F as defined above, and/or increasing an expression level of at least one gene selected from Group B, Group D and Group G, wherein Groups A to G are as defined above.

In the eleventh and twelfth inventions of the present invention, as the compound possessing an activity for controlling gene expression to substantially the same extent to that of compounds of (i) or (ii) mentioned above for at least one gene, there is exemplified a substance obtained by the screening method of any one of the first to third inventions of the present invention.

## BEST MODE FOR CARRYING OUT THE INVENTION

In one embodiment of the method of screening a biologically active substance of the present invention, a compound having the structure represented by the above-mentioned formula (I) is used. One example of the compound includes L-glycero-1,5-epoxy-1αβ,6-dihydroxy-cis-hex-3-en-2-one (DGE).

The compound can be obtained by treating (keeping) a compound having 3,6-anhydrogalactose at a reducing end thereof, such as at least one compound selected from the group consisting of agarobiose, κ-carrabiose and a compound having 3,6-anhydrogalactose at a reducing end thereof under neutral to alkaline conditions. In addition, the compound can also be obtained by subjecting the compound having 3,6-anhydrogalactose at a reducing end thereof to acid hydrolysis at a pH of less than 7 and/or enzymolysis, and subsequently treating (keeping) the resulting acid hydrolyzate and/or enzymolyzate under neutral to alkaline conditions.

The above-mentioned enzymolysis includes, for instance, degradation of agarose by using one compound having 3,6-anhydrogalactose at a reducing end thereof by using α-agarase. The conditions for carrying out the enzymolytic reaction are not particularly limited, and may be according to known conditions for used enzymes.

In the alkali treatment of the compound having 3,6-anhydrogalactose at a reducing end thereof, such as at least one compound selected from the group consisting of agarobiose, κ-carrabiose and a compound having 3,6-anhydrogalactose at a reducing end thereof, at a pH exceeding 7, the kinds of an alkali in which the compound is dissolved or suspended are not particularly limited. There can be used an inorganic base such as sodium hydroxide, potassium hydroxide, calcium hydroxide or ammonia, or an organic base such as Tris, ethylamine or triethylamine. The concentration of the alkali is not -particularly limited. The alkali can be used in a concentration of preferably from 0.0001 to 5 N, more preferably from 0.001 to 1 N. In addition, the treatment temperature is preferably, but is not particularly limited to, from 0° to 200°C, more preferably from 20° to 130°C. The treatment time is preferably, but is not particularly limited to, several seconds to several days. The kinds and concentration of the alkali, the temperature and time during the treatment may be properly chosen depending upon the kinds of the above-mentioned compound used as a raw material and the amount of the desired compound represented by the formula (I) formed. In general, the pH may be exceeding 7. The formation of the compound represented by the formula (I) proceeds quickly at a higher alkali concentration than those at a lower alkali concentration, and at a higher temperature than those at a lower temperature.

For instance, the compound represented by the formula (I) is formed by preparing a solution of agarobiose or κ-carrabiose at a pH of 11.5, and keeping the solution at 37°C for 5 minutes.

The resulting alkali solution containing the compound represented by the formula (I) may be used after neutralization according to its purpose, or may be used as an acidic solution having a pH of less than 7.

As the purification means for the compound represented by the formula (I) of the present invention, there may be employed a known means of purification such as a chemical method or a physical method. For instance, the purification may be carried out by combining various purification methods such as gel filtration, fractionation using a molecular weight fractionation membrane, solvent extraction, and various chromatographies using ion exchange resin or the like.

For instance, a compound represented by the formula (I) in which X is $CH_2OH$ and Y is H is purified from the product obtained by treating agarobiose under neutral to alkaline conditions, and a compound represented by the

formula (I) in which X is H and Y is $CH_2OH$ is purified from the product obtained by treating κ-carrabiose under neutral to alkaline conditions.

The derivative of the compound represented by the formula (I) is not particularly limited, as long as the derivative is capable of showing the gene expression pattern to substantially the same extent as that of the above compound in a living body. For instance, there can be used a compound obtainable by reacting the compound represented by the formula (I) with an SH-group containing compound (for instance, cysteine, glutathione, or known derivatives thereof). Here, a process for preparing the compound obtainable by reacting the compound represented by the formula (I) with an SH-group containing compound includes a known process described in WO 99/64424.

The salt of the compound represented by the formula (I) or a derivative thereof is preferably those acceptable as medicaments, and can be converted by a known method.

In addition, DGE, the compound represented by the formula (I), is a compound which is generated when an agarooligosaccharide is taken into a living body. Therefore, when the agarooligosaccharide is administered to a living body, it is anticipated that the agarooligosaccharide causes the same living body reaction as that of DGE [*Jpn. J. Phycol. (Sorui)* 48:13-19, March 10, 2000]. Therefore, in the present invention, there can be also used at least one compound selected from the group consisting of 3,6-anhydrogalactose represented by the above formula (II), an aldehyde thereof, a hydrate thereof, and a 2-O-methylated product and a 2-O-sulfated product of the foregoing compounds; and/or at least one compound selected from the group consisting of soluble saccharide compounds having the above compound at reducing end (Here, an aldehyde or a hydrate of 3,6-anhydrogalactose, and a 2-O-methylated product and a 2-O-sulfated product of the foregoing compounds is exemplified by the compound described in WO 00/41579), the compound from which DGE is thought to be generated when administered to a living body in place of the compound represented by the formula (I). Examples of the above compound are not particularly limited. For instance, the compound is exemplified by agarooligosaccharides such as agarobiose, agarotetrose, agarohexose, and agarooctose.

[1] Method of Screening Biologically Active Substance of the Present Invention

DGE, which is one of the above-mentioned compound represented by the formula (I), possesses actions of mitigating the damage to which the living body is subjected and progressing the maintenance of homeostasis in a living body. It has been known that DGE possesses an activity for regulating an expression level for a gene in which a change in an expression level is observed in a living body subjected to stress as mentioned above, namely an activity (or action) for controlling gene expression, and this activity is shown via expression control of various kinds of genes in a living body. Furthermore, DGE has known to possess various biological activities, for instance, apoptosis-inducing activity, anticancer activity, anti-oxidative activity such as activity for suppressing active oxygen production, activity for suppressing lipid peroxide radical production, and activity for suppressing nitrogen monoxide production, anti-pathogenic microbial activity, anti-mutagenic activity, α-glycosidase inhibitory activity, immunomodulatory action, action for suppressing prostaglandin synthesis, antiinflammatory action, anti-allergic action, action for regulating cytokine production, anti-rheumatic action, anti-diabetic action, activity for suppressing proliferation of synovial cell, action for inducing heat shock protein production, and the like. Therefore, the biological activity as described above is thought to be exhibited by regulating the gene expression level by gene controlling action of DGE.

The method of screening a biologically active substance of the present invention provides a method of screening a substance other than DGE capable of showing a change in gene expression level to the same extent as that of DGE by using as an index a change in gene expression level based on the gene controlling action of DGE which are known to have various biologically active substances as described above. In other words, there is provided a method of screening a substance capable of showing at least the same gene expression pattern as that of DGE in a living body.

Since the substance obtained according to the present invention can exhibit an activity for controlling gene expression to the same extent to that of DGE, it is deduced that the substance possesses, for instance, biological activities to the same extent as that of DGE. Therefore, by using the compound, there can be provided a medicament, food, beverage or the like which is effective for treatment or prevention of a disease and/or symptom (for instance, a disease requiring the above-mentioned biological activities of DGE, such as cancer, inflammation, allergy and diabetes) to which the expression of the biological activity effectively contributes.

Also, a substance having unknown biological activities or the like, which are different from the biological activities of DGE can be obtained. From this viewpoint, the method of screening a biologically active substance of the present invention can be also understood as a method of screening a candidate substance for a biologically active substance.

In the method of screening a biologically active substance of the present invention, concretely, a substance possessing an activity for controlling gene expression to the same extent to that of DGE can be obtained by the following steps:

(1) loading to a living body either 1) a test substance or 2) a compound of the above-mentioned (i) and/or the above-mentioned (ii);
(2) determining an expression level of a gene in the living body of the above (1), and comparing resulting values between a case where 1) is loaded and a case where 2) is loaded; and
(3) selecting a test substance which causes a change in an expression level of at least one gene to substantially the same extent as that of the case where 2) is loaded.

Here, the term "a change in an expression level ... to substantially the same extent" is not necessarily intended to mean that the extent of the expression level is the same, but to mean that at least a change in the expression level pattern is the same.

Concretely, a gene expression level in a sample derived from a living body to which a test substance 1) is loaded, namely administered or orally taken (hereinafter referred to the same) is determined. On the other hand, a gene expression level in a sample derived from a living body to which a compound 2), for instance, the above-mentioned compound represented by the formula (I), especially DGE, is loaded is determined. Next, each of the gene expression levels and/or its change is compared to judge whether or not the actions for controlling gene expression in each of the test substance and DGE is similar. The substance having a similar action for controlling gene expression has a high possibility of possessing the biological activities to the same extent as that of DGE, so that it is expected that the substance can be used for the same purpose as that of DGE.

The above-mentioned action for controlling gene expression owned by DGE is not particularly limited. For instance, the action can be confirmed by comparing the gene expression state (expression pattern) between a case where DGE is loaded to a living body and a case where a living body is not loaded (control). In this case, the gene having a difference in expression levels therebetween is thought to be a gene to be controlled by DGE. Here, the action for controlling gene expression owned by DGE, as described in Example 1 described later, can be also confirmed via loading stress to a living body.

In the present specification, the living body is not particularly limited. The preferred living body includes an organism individual (excluding human) or cultured cell. For instance, when the organism individual is used, the gene expression state can be examined by taking an appropriate sample from the individual, for instance a part of a tissue, body fluid or the like.

The test substance usable in the screening method of the present invention is not limited by any means, and there can be used those derived from nature, those artificially synthesized, those obtained by artificially modifying a substance derived from nature, and the like. As these substances, any of those which are isolated and those containing plural substances can be used. For instance, appropriate separation procedures and the screening method of the present invention are combined to purify or isolate a useful substance.

In addition, by using the screening method of the present invention, an active component thereof can be specified from a composition possessing biological activities to substantially the same extent as that of the above-mentioned compound (i) or the above-mentioned compound (ii), for instance, from a composition derived from nature such as those derived from a plant, an animal, Basidiomycetes, or an microorganism. For instance, an active component can be specified by carrying out the method of screening a biologically active substance of the present invention by using the compound constituting the above-mentioned composition as a test substance.

In addition, the present invention encompasses the above-mentioned substance obtained by the screening method of the present invention, namely the substance having the gene controlling activity to the same extent as that of the above-mentioned compound (i) or the above-mentioned compound (ii). The above substance can be obtained by subjecting a test substance to the screening method of the present invention, and judging whether or not the substance possesses gene controlling activity as defined in the above method. In addition, the substance is exemplified by, for instance, a substance derived from a plant, a substance derived from Basidiomycetes, or a derivative thereof. The plant usable herein is exemplified by medicinal plants, marine algae, and the like, which have been known to have various biological activities.

Also, the substance derived from these origins is not particularly limited. The constituent thereof includes a substance containing a saccharide, for instance, polysaccharides, degradation products thereof or derivatives thereof. Among them, those substances having a molecular weight of 100 to 2000 or so are preferable. Further, among them, a substance possessing a gene controlling activity other than the substance used as a control (for instance, DGE) according to the screening method of the present invention can be selected. The above-mentioned substance containing a saccharide as a constituent is especially preferably a substance having 3,6-anhydrogalactose in its molecule. Here, the degradation product, the derivative, and the originating substance are not particularly limited, as long as they can show the gene expression pattern of the substantially same extent in a living body.

The comparison of the gene expression states can be carried out by a known method, for instance, the determination of the level of the above gene product (polypeptide), or the determination of the amount of mRNA transcribed from the above gene. The method of determining the amount of mRNA is more effective for the above-mentioned purposes, and is preferable.

For instance, it is preferable that the level of the polypeptide is determined by using an antibody capable of specifically binding to the polypeptide or a fragment thereof. The antibody may be any of polyclonal antibodies and monoclonal antibodies. Furthermore, the antibody may be those antibodies which are modified by known techniques, and the derivatives of the antibodies, for instance, Fab fragment, single-strand antibody and the like. These antibody and derivative thereof or the like can be produced in accordance with a known method (for instance, see *Current Protocols in Immunology*, published by John Wiely & Sons, Inc., 1992, edited by John E. Coligan). The method for determining the level of the polypeptide includes, for instance, known enzyme immunoassay, fluoroimmunoassay, luminescent immunoassay, and the like. Further, the above-mentioned antibody or the like may be subjected to various kinds of labeling in order to facilitate the detection in the above-mentioned method for determining the level of the polypeptide.

The method for determining mRNA is not particularly limited, and a known method such as RT-PCR method, Northern hybridization method, dot blot hybridization method, a hybridization method using a DNA array, a DNA microbeads method, or the like can be employed. The method using a DNA array is especially preferable, for the above-mentioned purpose, because the expression states for numerous genes can be examined with a very little amount of a sample.

The "DNA array" as referred to herein is one in which a gene or a DNA fragment derived from the gene is immobilized on a support in a given region (position), and encompasses, for instance, those referred to as DNA chips. In addition, one in which gene or a DNA fragment derived from the gene is immobilized on a support in a high density is referred to as DNA microarray.

The support of the DNA array is not particularly limited, as long as it can be used for hybridization, and slide glass, silicon chip, nitrocellulose or nylon membrane or the like can be usually used. The surface of the support may be any of those in which a single-stranded DNA or double-stranded DNA can be immobilized by covalent bonding or non-covalent bonding and those having a hydrophilic or hydrophobic functional group on the surface of the support without being particularly limited thereto. For instance, there can be preferably used those having hydroxyl group, amino group, thiol group, aldehyde group, carboxyl group, an acyl group or the like without being particularly limited thereto. These functional groups may exist to give the surface properties of the support itself, or may be introduced by surface treatment. The surface-treated product as described above includes, for instance, those obtained by treating glass with a commercially available silane coupling agent such as an aminoalkylsilane; those obtained by treating glass with a polycation such as polylysine or polyethyleneimine; and the like. Also, some of the slide glass subjected to these treatments are commercially available.

The gene or a DNA fragment thereof, which is immobilized to the support, is not particularly limited, and includes, for instance, genomic DNA library and cDNA library, and a DNA amplified with these libraries as a template by a known nucleic acid amplification method, for instance, PCR (polymerase chain reaction) method, LCR (ligase chain reaction) method, SDA (strand displacement amplification) method, ICAN (isothermal and chimeric primer-initiated amplification of nucleic acids) method (described in WO00/56877), and the like. The reaction conditions in the nucleic acid amplification are not particularly limited. In addition, there can be used a DNA obtained by subjecting amplified RNA to reverse transcription by transcription-based amplification system (TAS) method using RNA as a template, self-sustained sequence replication (3SR) method, NASBA (nucleic acid sequence-based amplification) method, TMA (transcription-mediated amplification) method or Qβ replicase method.

The procedure for immobilization to a support can be carried out by using a commercially available apparatus for preparing a DNA array, for instance, an apparatus for preparing DNA chip manufactured by Affymetrix. By using the apparatus, a DNA array (DNA microarray) in which the genes are aligned and immobilized in a high density can be prepared.

In the DNA array usable in the present invention, those obtained by immobilizing an arbitrary gene or a DNA fragment derived from the gene can be used. It is preferable to use an array to which a gene encoding a protein possessing a function associated with maintenance of homeostasis in a living body or a DNA fragment derived from the gene is immobilized. Also, there are made commercially available a DNA microarray in which a fragment of various genes is immobilized (IntelliGene Human Cytokine CHIP or the like manufactured by Takara Shuzo Co., Ltd.), which may be also used. Also, the selection of the gene or a DNA fragment derived from the gene which is to be immobilized to the DNA array can be carried out by the DNA microbeads method described in Example 3 given later, the subtraction method, the differential display method or the like, whereby selecting a gene which is found to show a change in expression levels between a living body subjected to loading of stress to a living body, for instance, a drug which causes hindrances in the maintenance of homeostasis in a living body, such as phorbol myristate acetate and lipopolysaccharide, and a living body without being subjected to loading.

By using the DNA array as described above, the amount of numerous kinds of the nucleic acid molecules contained in a nucleic acid sample can be simultaneously determined. Also, there is an advantage such that the determination can be made with a small amount of a nucleic acid sample. For instance, mRNA in a sample is labeled, or

labeled cDNA is prepared with mRNA as a template, and the hybridization is carried out between the labeled product and the DNA array, whereby a plural mRNAs transcribed in the sample can be simultaneously detected and their expression levels can be also determined.

The difference in the gene expression levels in a living body in a case where DGE is loaded and a case where DGE is not loaded can be examined by, for instance, comparing the gene expression patterns in both cases as follows.

Concretely, the results for each of hybridization of a nucleic acid in a sample derived from an organism to which DGE is loaded (DGE-loaded sample) with the DNA array and hybridization of a nucleic acid in a sample derived from a living body to which DGE is not loaded (control sample) with the DNA array are compared, whereby a gene having different expression levels in both cases can be detected. For instance, an appropriate signal is detected depending upon the label used in a DNA array hybridized with a nucleic acid in the sample, wherein the nucleic acid is labeled by any methods, and thereafter each gene on the array are compared for the expression levels in the DGE-loaded sample and in the control sample. It is preferable that each of nucleic acids in the DGE-loaded sample and the control sample is previously labeled by a different method, whereby the difference in the gene expression level in the DGE-loaded sample and the gene expression level in the control sample can be compared by competitive hybridization on the same DNA array by using a multi-wavelength detectable fluorescence analyzer which is capable of detecting plural labels, for instance two kinds of fluorescence. Specifically, the difference in the gene expression of both the samples can be detected as a difference in the color of the signals and in intensities by carrying out hybridization of a mixture of equivalent amount of both samples with the DNA array. As a result, the gene having a significant difference in the intensities of the signals obtained between both the samples is specified as the gene to be controlled in its expression by DGE.

More concretely, the difference in the gene expression levels between the DGE-loaded sample and the control sample can be examined by the following steps.

mRNA is prepared from a sample derived from an organism to be compared with each other, for instance, from a cell subjected to different treatments, and mRNA in the sample, cDNA derived from the mRNA, or a nucleic acid transcribed or amplified by the cDNA is labeled. As the labeling substance usable in labeling, there can be used substances such as radioisotopes, fluorescent substances, chemiluminescence substances, substances having chromophores, and the like. For instance, the fluorescent substance includes Cy2, FluorX, Cy3, Cy3.5, Cy5, Cy5.5, Cy7, fluorescein isothiocyanate (FITC), texas red, Rhodamine, and the like. In addition, it is desired that each of the DGE-loaded sample and the control sample is labeled with a different fluorescent substance from each other using two or more fluorescent substances, preferably two fluorescent substances from the viewpoint of simultaneous detection. In addition, the labeling can be carried out, for instance, by co-existence of a labeled nucleotide during cDNA synthesis from mRNA or during transcription and amplification reaction from cDNA, besides the method of chemically labeling mRNA in the sample, cDNA derived from the mRNA or a nucleic acid transcribed or amplified from the cDNA.

Next, hybridization of the above-mentioned labeled nucleic acid with the DNA array, to which a nucleic acid corresponding to an appropriate gene or a fragment thereof is immobilized, is carried out. The hybridization may be carried out by a known method, and its conditions may be appropriately selected for those suitable for the usable DNA array and labeled cDNA. For instance, the hybridization can be carried out under the conditions described in *Molecular Cloning, A Laboratory Manual,* Second Edition, 9.52-9.55 (1989).

The signals from the labels obtained by the above-mentioned hybridization are compared, whereby a gene which has a significant difference in the signal intensities between the samples can be detected. Concretely, signal intensities in radioactivity, fluorescence, luminescence and the like for the array which is subjected to hybridization with the sample of nucleic acid labeled by the above-mentioned method are detected with a specialized detector, for instance, a chromatoscanner, image analyzer or the like, whereby the gene showing a significant changes in its expression levels can be detected from the difference in the signal intensities. The method for detecting the label may be selected appropriately depending upon the kinds of the usable labeling substances.

By labeling the nucleic acid derived from each sample (mRNA, cDNA derived from the mRNA or a nucleic acid transcribed or amplified by the cDNA) with a labeling substance which can be distinguished from each other, the difference in the gene expression levels in both the samples can be compared in a single hybridization. For instance, when the above-mentioned Cy3 and Cy5 are used as labeling substances, the gene expression levels in both the samples can be determined on a single DNA array by scanning at a wavelength of 532 nm for Cy3 and at a wavelength of 635 nm for Cy5.

Thus, the expression levels of numerous kinds of genes between different samples can be compared using a very little amount of the samples.

Here, in order to determine the difference in the gene expressions between the DGE-loaded sample and the control sample, the amounts of mRNA of both the samples and the difference in intensities between the used fluorescent substances need to be corrected. The correction can be carried out by a known method by using a standard gene with a relatively little expression variation (preferably housekeeping gene).

Also, in the present specification, a case where the difference in gene expression levels is judged to be significant is a case where the ratio of the gene expression level in the DGE-loaded sample to the gene expression level in the control sample, namely a relative expression ratio (gene expression level in DGE-loaded sample/gene expression level in control sample) is 1.7 or more or 0.6 or less. Here, it is judged as a gene of which expression level is increased when the relative expression ratio is 1.7 or more, and as a gene of which expression level is decreased when the relative expression ratio is 0.6 or less.

Furthermore, the difference in the gene expression levels between the DGE-loaded sample and the control sample can be carried out by a known DNA microbeads array method. According to this method, all the expressible genes can be analyzed regardless of the expression level or being known or unknown.

The gene expression states are compared according to the above-mentioned method between a living body that is administered or orally taken with DGE and a control to which DGE is not given, whereby the gene which is controlled by DGE for its expression can be confirmed. The genes to which DGE can affect through its action for controlling gene expression, which are confirmed as described above, include, for instance, the genes listed as Groups A to G shown in Tables 1 to 3 given later.

An organism is subjected to various stresses caused by external factors such as change in living environment and internal factors such as viral and bacterial infections. The stress includes, for instance, high-temperature and low-temperature environments, starvation state, exposure to chemical substances, and the like. The chemical substance as referred to herein includes, for instance, phorbol myristate acetate (TPA), lipopolysaccharide (LPS), phytohemagglutinin (PHA), okadaic acid and the like. Among them, TPA and LPS cause inflammation by administration to a living body, and have been used for the purpose of artificially producing inflammation model by utilizing its action.

As a consequence of being subjected to the stress as mentioned above, there are changes in expression of various genes in a living body. Among them, there are a group of genes of which expressions are induced or enhanced as a consequence of the stress, thereby causing bad influence to a living body (for instance, genes belonging to Group A and Group E); or a group of genes of which expressions are induced or enhanced in order to remove the bad influence by the stress, or expressions are the same extent as that without the stress (for instance, genes belonging to Group B and Group G). If stress leads to a high expression state in the genes belonging to Group A and Group E, and the state in which the expression state does not decrease continues, it is thought to lead to incidence of various diseases. Therefore, leading to the state where expression of genes belonging to Group A and Group E is difficult to increase when subjected to stress results in prevention of various diseases. Also, promptly lowering the increased expression of genes belonging to Group A and Group E when subjected to stress result in prevention, treatment or inhibition of worsening of various diseases. On the other hand, the genes belonging to Group B and Group G are a group of genes of which expressions are increased to avoid the state of stress. The state of stress can be promptly avoided by expressing the group of genes mentioned above at a higher level when subjected to stress, whereby probably leading to prevention, treatment or inhibition of worsening of various diseases caused by the stress.

In addition, as a consequence of the stress, there are some groups of genes of which expressions are decreased or of the same extent as that without stress in order to remove the bad influence of the stress (for instance, genes belonging to Group C and Group F), and other groups of genes of which expressions are decreased and which give bad influences to a living body (for instance, genes belonging to Group D). The genes belonging to Group C is a group of genes of which expressions are decreased in order to avoid the state of stress. The state of stress can be promptly avoided by further suppressing the expression level of the group of genes described above when subjected to stress, whereby probably leading to prevention, treatment or inhibition of worsening of various diseases caused by the stress. On the other hand, if stress leads to a low expression state in the genes belonging to Group D, and the state in which expression does not increase continues, it is thought to lead to incidence of various diseases. Therefore, increasing the expression of the genes belonging to Group D when subjected to stress results in prevention of various diseases. In addition, promptly increasing the decreased expression of the genes belonging to Group D when the living body is subjected to stress also results in prevention, treatment or inhibition of worsening of various diseases.

The genes belonging to Group A, Group C, Group E and Group F of which expressions are suppressed (decreased) by DGE, and the genes belonging to Group B, Group D and Group G of which expressions are accelerated (increased) by DGE, which are elucidated according to the present invention, are shown in Tables 1 to 3.

Table 1

| Gene Name | GenBank Accession No. |
|---|---|
| Group A | |
| Bone morphogenetic protein-6 (BMP-6) | NM_001718 |
| Chemokine receptor-1 (CCR-1) | NM_001295 |
| Hepatocyte growth factor (HGF) | X16323 |
| Intercellular adhesion molecule-1 (ICAM-1) | NM_000201 |

Table 1 (continued)

| Gene Name | GenBank Accession No. |
|---|---|
| Group A | |
| Interleukin-1β (IL-1β) | X02532 |
| Interleukin-16 (IL-16) | M90391 |
| Leukemia inhibitory factor (LIF) | NM_002309 |
| Osteopontin | NM_000582 |
| Tumor necrosis factor-α (TNF-α) | X02910 |
| Neuropilin-1 | NM_003873 |
| signal transducer and activator of transcription 6 (STAT6) | AF067575 |
| Gene containing Homo sapiens cDNA: FLJ22298 fis, clone HRC04637 | AK025951 |
| Group B | |
| Heme oxygenase-1 (HO-1) | NM_002133 |
| Thioredoxin reductase-1 | AF106697 |
| putative translation initiation factor (SUI1) | AF083441 |
| Homo sapiens hypothetical protein (HSPC014) | NM_015932 |

Table 2

| Gene Name | GenBank Accession No. |
|---|---|
| Group C | |
| N-myc downstream regulated gene 1 (NDRG1) | NM_006096 |
| adenosine deaminase, RNA specific (ADAR) | NM_015841 |
| splicing factor 3b, subunit2 (SF3B2) | NM_006842 |
| interleukin enhancer binding factor 3 (ILF3) | NM_004516 |
| valyl-tRNA synthetase 2 (VARS2) | NM_006295 |
| Gene containing Nucleotide Sequence Shown in SEQ ID NO: 12 of Sequence Listing | None |
| Group D | |
| eukaryotic translation elongation factor 1 alpha 1 (EEF1A1) | NM_001402 |
| ribosomal protein L17 (RPL17) | NM_000985 |
| ribosomal protein L24 (RPL24) | NM_000986 |
| splicing factor, arginine/serine-rich 2 (SFRS2) | NM_003016 |
| tumor protein, translationally-controlled 1 (TPT1) | NM_003295 |
| ubiquinol-cytochrome c reductase binding protein (UQCRB) | NM_006294 |
| ribosomal protein S15a (RPS15A) | NM_001019 |
| ribosomal protein L27 (RPL27) | NM_000988 |

Table 3

| Gene Name | GenBank Accession No. |
|---|---|
| Group E | |
| Interleukin-2 receptor α (IL2RA) | X01057 |
| Interleukin-6 (IL6) | X04430 |
| CD166 | Y10183 |
| Interferon regulatory factor-1(IRF1) | X14454 |
| Interleukin-15 receptor α (IL15RA) | U31628 |

Table 3 (continued)

| Gene Name | GenBank Accession No. |
|---|---|
| Group E | |
| Nuclear factor kappa-b p105 subunit (NFKB1) | M58603 |
| Caspase-1(CASP1) | M87507 |
| Interleukin -1β (IL1B) | M15330 |
| Tumor necrosis factor-α (TNF-α) | X02910 |
| Gro1 oncogene (GRO1) | X54489 |
| Interleukin -1α (IL1A) | M28983 |
| Inhibin βA (INHBA) | J03634 |
| Interleukin-7 receptor (IL7R) | M29696 |
| Precursor B cell colony enhancing factor (PBEF) | U02020 |
| Liver and activation regulated chemokine (LARC) | U64197 |
| Interleukin-8 (IL8) | M26383 |
| Group F | |
| Colony-stimulating factor-1 receptor (CSF1R) | X03663 |
| Chemokine (C-X-C motif) receptor-4 (CXCR4) | AF147204 |
| Endoglin (ENG) | NM_000118 |
| Group G | |
| Heme oxygenase-1 (HMOX1) | Z82244 |

The action for controlling gene expression is examined for a test substance in the same manner as in DGE described above, and the action is compared with that of DGE, whereby a substance having an activity for controlling gene to the same extent as that of DGE can be selected. Here, in the comparison of a change in gene expression level of a case where a test substance is loaded with that of a case where DGE is loaded, concretely in the comparison of the expression patterns, at least one of the genes may be compared, and it is preferable that plural gene expression patterns are compared, from the viewpoint of finding a more useful substance. Although the present invention is not particularly limited, for instance, the expression patterns of preferably two or more kinds of genes, more preferably four or more kinds of genes, still more preferably seven or more kinds of genes are compared to select a substance having the desired activity for controlling gene.

In a preferred embodiment, a comparison may be made for at least one gene selected from those of Groups A to G shown in Tables 1 to 3 mentioned above. More preferably, the expression patterns of at least one gene selected from Group A, Group C, Group E and Group F and at least one gene selected from Group B, Group D and Group G (total of two or more genes), still more preferably the expression patterns of at least one gene selected from each group of Groups A to G (total of seven or more genes), are compared.

In addition, on the bases of the findings, according to the present invention, there is provided another embodiment of a method of screening a biologically active substance of the present invention as described below. Specifically, according to the present invention, there is provided a method of screening a biologically active substance, which comprises the following steps:

(1) loading a test substance to a living body;
(2) determining an expression level of a gene in the living body of the above (1) and an expression level of a gene in the living body without the loading, and comparing resulting values therebetween; and
(3) selecting a test substance which decreases an expression level of at least one gene selected from Group A, Group C, Group E and Group F in Tables 1 to 3, and/or increases an expression level of at least one gene selected from Group B, Group D and Group G in Tables 1 to 3.
According to this method, there can be conveniently obtained a substance possessing an activity for controlling gene expression to the same extent to that of DGE.

In addition, there is provided another embodiment of a method of screening a biologically active substance of the present invention as described in further detail below. Specifically, according to the present invention, there is provided a method of screening a biologically active substance, which comprises the following steps:

(1) loading stress to a living body, and loading a test substance with stress to a living body, respectively;
(2) determining each of expression levels of a gene in the living body of (1) and in a normal living body without any of the loading; and
(3) comparing the expression levels of a gene obtained in the above (2) between the living body of the above (1) and the normal living body, and selecting a test substance showing at least one gene expression pattern selected from the gene expression patterns of the followings (a) to (g):

(a) an expression level of at least one gene selected from Group A as defined above increases when stress is loaded to a living body, and decreases when a test substance and stress are loaded to a living body, as compared to that of the normal living body;
(b) an expression level of at least one gene selected from Group B as defined above increases when stress is loaded to a living body, and further increases when a test substance and stress are loaded to a living body, as compared to that of the normal living body;
(c) an expression level of at least one gene selected from Group C as defined above decreases when stress is loaded to a living body, and further decreases when a test substance and stress are loaded to a living body, as compared to that of the normal living body;
(d) an expression level of at least one gene selected from Group D as defined above decreases when stress is loaded to a living body, and increases when a test substance and stress are loaded to a living body, as compared to that of the normal living body;
(e) an expression level of at least one gene selected from Group E as defined above increases when stress is loaded to a living body, and decreases when a test substance and stress are loaded to a living body, as compared to that of the normal living body;
(f) an expression level of at least one gene selected from Group F as defined above is the same extent to that of the normal living body when stress is loaded to a living body, and decreases when a test substance and stress are loaded to a living body, as compared to that of the normal living body; and
(g) an expression level of at least one gene selected from Group G as defined above is the same extent to that of the normal living body when stress is loaded to a living body, and increases when a test substance and stress are loaded to a living body, as compared to that of the normal living body. According to this method, a substance possessing an activity for controlling gene to the same extent as that of DGE can be conveniently obtained.

Here, in the above-mentioned two of another embodiments, the action for controlling gene expression of the test substance can be examined in the same manner as that of DGE mentioned above. Also, the load of stress to a living body is carried out by loading various stresses as mentioned above to a living body, which is preferably carried out by using a chemical substance. Among them, the treatment with TPA (phorbol myristate acetate) or the treatment with LPS (lipopolysaccharide) is more preferred. Here, the treatment refers to administration or oral intake.

TPA has been known as a promoter of carcinogenesis, and carcinogenic action caused by inflammation induction of a living body has been known as its action besides a direct mutation action. Concretely, TPA causes freeing of an inflammatory mediator such as NO or prostaglandin $E_2$ from an inflammatory cell such as macrophage.

LPS is a cytokine-inducing factor, and causes various influences such as fervescence, lethal effects and lowering in blood pressure in a living body, and its action is not a direct action of LPS itself, but an action of inflammatory cytokine or the like, produced by a host cell centering about macrophage by LPS.

The above-mentioned Groups A to D are a group of genes which are confirmed to fluctuate when TPA is used as stress, and the above-mentioned Groups E to G are a group of genes which are confirmed to fluctuate when LPS is used. DGE suppresses inflammation induced by TPA or LPS. Therefore, concretely, if the compound showing any of the above-mentioned gene expression patterns (a) to (d) is selected when treated with TPA as stress in the above-mentioned step, there can be expected to obtain a compound having effects such that the inflammation induced by the same mechanism as that of the inflammation induced by TPA is treated or prevented. Also, if the compound showing any of the above-mentioned gene expression patterns (e) to (g) is selected when treated with LPS as stress, there can be expected to obtain a compound having effects such that the inflammation induced by the same mechanism as that of the inflammation induced by LPS is treated or prevented.

Even if a substance has slightly different action for controlling gene expression, there is a possibility that the substance possess some of the biological activities owned by DGE. Such a substance can be used according to its purpose as a substitute substance for DGE.

Also, in another embodiment, there can be provided a kit suitably usable for the method of screening a biologically active substance of the present invention. The kit may at least contain primers and/or a probe for detecting mRNA or a fragment thereof expressed from at least one gene, for instance, selected from Groups A to G in Tables 1 to 3, or an antibody or a fragment thereof against a polypeptide or a fragment thereof, wherein the polypeptide is

encoded by the gene.

[2] DNA Array of the Present Invention

Also, the present invention provides a DNA array usable for the above-mentioned method of screening a biologically active substance, wherein at least two genes selected from the genes of Groups A to G as defined above or fragments thereof are immobilized on a support at a given position. The DNA array of the present invention is an array in which genes of which expression is controlled by the above-mentioned DGE, or DNA fragments derived from the gene are immobilized on a support at a given position. In other words, the DNA corresponding to each of the genes or fragments thereof are arranged in given positions on the support. Therefore, when such an array is used, there can immediately be known from which gene of DNA or fragments thereof a signal is derived on the basis of the position of the detected signal. The array is useful for screening the biologically active substance according to the above [1], and genetic pathogenic analysis in a disease for which administration or intake of DGE is effective.
The genes or DNA fragments thereof to be immobilized on the above-mentioned DNA array are not particularly limited, as long as they are genes or DNA fragments thereof of which expression is controlled by stress to a living body or DGE, or those genes or fragments containing these. For instance, the genes or DNA fragments thereof to be immobilized can be selected by using the DNA microbeads method described in Example 3 mentioned later. Also, there may be also contained a gene other than the gene of which expression is controlled by stress to a living body or DGE, for instance, a gene usable as a negative control or the like. Also, the production of the array may be carried out by a known method. For instance, the apparatus for preparing a DNA array as described in the above [1] can be used.
In the DNA array of the present invention, the genes or DNA fragments thereof may be immobilized in either form of single-strand or double-strand. Also, they may be any of polynucleotides and oligonucleotides. In addition, their production method is not particularly limited, which may be those chemically synthesized, those derived from nature, those enzymatically synthesized, or a combination of these. Here, the strand length of the gene or a DNA fragment thereof is not particularly limited. For instance, it is desired that the strand length is about 20 bases long to about 1 kilo bases long. Also, those having a strand length shorter or longer than that specified above may be used, as long as they are capable of specifically hybridizing with a nucleic acid in any sample to be tested upon the hybridization.
The support for the array and the density for immobilizing DNA are not particularly limited, and there may be selected those appropriate for the purpose and the number of genes to be immobilized. For instance, the support for the DNA array includes those mentioned above, and is not particularly limited as long as the support can be used for hybridization. Usually, there may be employed slide glass, silicon chip, nitrocellulose or nylon membrane, or the like. Also, when a large number of genes are immobilized or when an array suitable for a very little amount of the sample is to be prepared, a microarray in which genes are immobilized to a support in a high density can be suitably used. As the microarray, those in which nucleic acids, especially DNAs, are immobilized in a density of 100 dots/$cm^2$ or more are preferable. In addition, the shape is not particularly limited, and there can be appropriately selected those having flat plate-shape, disc-shape, tape-shape, or the like.

[3] Gene Controlling Agent and Method of Controlling Gene of the Present Invention

Also, regarding the method of controlling a gene expression in a living body, the present invention provides, but not particularly limited to, a method of controlling (regulating) a gene expression for a gene of which expression level is changed in a living body subjected to a stress, thereby maintaining homeostasis in a living body, and a gene controlling agent for the method.
For instance, it is thought that the influence in a body subjected to a stress as mentioned above can be eliminated by properly controlling the gene expression in the living body. For this purpose, it is effective to use a compound capable of controlling the gene, for instance, those listed in Tables 1 to 3, or a composition comprising the compound as an effective ingredient, concretely the gene controlling agent provided by the present invention. Therefore, the gene controlling agent is especially preferable for mitigating or preventing stress.
As the compound which can be contained as the effective ingredient in the above-mentioned gene controlling agent, there can be used, for instance, DGE, a derivative of DGE and/or a salt thereof, and furthermore a compound possessing an activity for controlling gene expression to the same extent as that of DGE, a derivative thereof and/or a salt thereof. The derivative or the like of the compound can be prepared in the same manner as the derivative or the like of the compound represented by the formula (I). As the compound possessing an activity for controlling gene expression to the same extent as that of DGE, there can be preferably used a substance obtained by the screening method described in the above [1].
The gene controlling agent can be prepared in accordance with a known method for producing a medicament. For instance, the above-mentioned effective ingredient is mixed with a known pharmacologically acceptable liquid or solid vehicle, and further a solvent, a dispersant, an emulsifier, a stabilizer, a lubricant, or the like which has been

known is optionally added thereto, to give a liquid agent such as a general liquid agent, a suspension agent, or an emulsion agent, or a solid agent such as a tablet, a granule, a powder or a fine powder.

The above-mentioned vehicle can be selected depending upon the administration form and preparation form of the gene controlling agent of the present invention. In the case of an orally administered preparation, there can be preferably utilized, for instance, starch, lactose, mannitol, carboxymethyl cellulose, or the like. On the other hand, in the case of a non-orally administered preparation, there can be preferably utilized distilled water for injection, physiological saline, soybean oil, propylene glycol or the like.

The content of the above-mentioned effective ingredient in the gene controlling agent of the present invention cannot be absolutely determined because the content changes depending upon the administration form and the administration method of the gene controlling agent. The content is not particularly limited as long as the amount is such that the above-mentioned effective ingredient is administered in an amount that can give the desired effects of the present invention.

The gene controlling agent of the present invention can be administered orally or non-orally via an appropriate administration route appropriate for the preparation form. In the case of a non-orally administered preparation, the injection can be carried out, for instance, intravenously, intramuscularly, subcutaneously, intracutaneously, or the like. The dose for the gene controlling agent of the present invention may be properly determined within the range in which the desired effects of the present invention can be obtained depending upon its preparation form, administration method, purpose of use, or age, body weight, or symptom of the patient to be administered or the like.

Concretely, the gene controlling agent of the present invention is exemplified as follows:

(1) a gene controlling agent comprising a compound possessing an action of decreasing an expression level of at least one gene selected from Group A, Group C, Group E and Group F as defined in Tables 1 to 3, and/or increasing an expression level of at least one gene selected from Group B, Group D and Group G as defined in Tables 1 to 3;
(2) a gene controlling agent comprising a compound possessing an action of decreasing an expression level of at least one gene selected from Group A, Group C, Group E and Group F as defined in Tables 1 to 3, and/or increasing an expression level of at least one gene selected from Group B, Group D and Group G as defined in Tables 1 to 3, in a living body to which stress is loaded;
(3) a gene controlling agent comprising a compound possessing an action of decreasing an expression level of at least one gene selected from Group A and Group E as defined in Tables 1 to 3 in a living body to which stress is loaded, wherein the expression level of at least one gene is increased by the stress;
(4) a gene controlling agent comprising a compound possessing an action of increasing an expression level of at least one gene selected from Group B and Group G as defined in Tables 1 to 3 in a living body to which stress is loaded, wherein the expression level of at least one gene is increased by the stress;
(5) a gene controlling agent comprising a compound possessing an action of decreasing an expression level of at least one gene selected from Group C and Group F as defined in Tables 1 to 3 in a living body to which stress is loaded, wherein the expression level of at least one gene is decreased by the stress; and
(6) a gene controlling agent comprising a compound possessing an action of increasing an expression level of at least one gene selected from Group D as defined in Tables 1 to 3 in a living body to which stress is loaded, wherein the expression level of at least one gene is decreased by the stress.

Further, according to the present invention, there are provided:

a method of controlling a gene comprising administering to a living body either at least one compound selected from the group consisting of the above-mentioned compounds of (i) and (ii) as defined in Tables 1 to 3 and a compound possessing an activity for controlling gene expression to substantially the same extent to that of the compounds for at least one gene, or a composition comprising the compound, thereby decreasing an expression level of at least one gene selected from Group A, Group C, Group E and Group F as defined in Tables 1 to 3, and/or increasing an expression level of at least one gene selected from Group B, Group D and Group G as defined in Tables 1 to 3; and
a method of controlling a gene comprising administering to a living body to which stress is loaded either at least one compound selected from the group consisting of the above-mentioned compounds of (i) and (ii) and a compound possessing an activity for controlling gene expression to substantially the same extent to that of the compounds for at least one gene, or a composition comprising the compound, thereby decreasing an expression level of at least one gene selected from Group A, Group C, Group E and Group F as defined in Tables 1 to 3, and/or increasing an expression level of at least one gene selected from Group B, Group D and Group G as defined in Tables 1 to 3.

Here, a living body refers to a mammal, especially human. Also, as the compound possessing an activity for

controlling gene expression to substantially the same extent for at least one gene, there can be preferably used the substances obtained according to the screening method described in the above [1].

According to the method of controlling gene, the expressions of genes shown in Tables 1 to 3 can be preferably controlled, whereby stress to which a living body is subjected can be mitigated and/or avoided.

The administration method, the dose and the like of the compound administered as the effective ingredient or the composition comprising the compound may be, for instance, the same as those of the above-mentioned gene controlling agent.

According to the method of controlling the gene of the present invention, the expression of a group of genes causing worsening effects to a living body when subjected to stress can be suppressed, and further expression of a group of genes involved in avoidance of stress state is enhanced, whereby various diseases can be prevented, treated, or inhibited from becoming worsened.

The present invention will be more concretely described below by means of Examples, without limiting the present invention thereto.

Example 1

(1) Preparation of RNA

HL-60 cells (ATCC CCL-240) were suspended in RPMI 1640 medium (manufactured by Bio Whittaker, 12-702F) containing 10% fetal bovine serum (manufactured by JRH Biosciences) and 1.3% dimethyl sulfoxide, and the cells were cultured at 37°C for one week in the presence of 5% carbon dioxide gas to induce differentiation to neutrophile-like cells.

The cells obtained as described above were collected by centrifugation, and suspended in 10% fetal bovine serum-containing RPMI 1640 medium so as to have a concentration of $10^6$ cells/mL, and 10-mL portions of the cells were added to a 10-cm petri dish. In each petri dish, there was set a group in which 100 μL of a 4 mM DGE aqueous solution was added thereto, and total RNA was collected 4 hours thereafter; a group in which 100 μL of a 100 μg/mL dimethyl sulfoxide solution of phorbol myristate acetate (TPA, manufactured by Gibco, 13139-019) was added thereto, and total RNA was collected 4 hours thereafter; or a group in which 100 μL of a 4 mM DGE aqueous solution was added thereto, and the cells were cultured for 6 hours, and thereafter, additional 10 μL of 100 μg/mL TPA dimethyl sulfoxide solution was added thereto, and total RNA was collected 4 hours thereafter. Also, as the negative control, there was set a group in which 100 μL of water was added to a petri dish, and total RNA was collected 4 hours thereafter. Here, RNA was prepared by using RNeasy Mini Kit (manufactured by QIAGEN, 74104) in accordance with the instruction for the kit.

(2) Synthesis of cDNA

Twenty-five micrograms of each of the total RNAs obtained as described above and 100 pg of λ poly $A^+$ RNA-A (manufactured by Takara Shuzo Co., Ltd., TX802) were subjected to synthesis reaction of fluorescence-labeled cDNA by using RNA Fluorescence Labeling Core Kit (manufactured by Takara Shuzo Co., Ltd., TX807) and 1 mM FluoroLink Cy3-dUTP or Cy5-dUTP (manufactured by Amersham-Pharmacia Biotech, PA53022(Cy3), PA55022(Cy5) in accordance with the instruction for the kit and the resulting cDNA was purified. The solutions of fluorescence-labeled DNA were mixed in the combination of Table 4, and the mixture was subjected to a chloroformisoamyl alcohol (24:1) extraction. DNA was collected by ethanol precipitation with 2 μL of 5 × Competitor I (Human) (manufactured by Takara Shuzo Co., Ltd., TX808) added. The resulting DNA was dissolved in 10 μL of a hybridization solution (a solution containing 6 × SSC, 0.2% SDS, 5 × Denhardt's solution, 0.1 mg/mL denatured salmon sperm DNA solution passed through a filter having a size of 0.22 μm), and subjected to hybridization.

Table 4

| | |
|---|---|
| Combination 1 | Negative Control (Cy3-Labeled) and Group with Addition of DGE (Cy5-Labeled) |
| Combination 2 | Negative Control (Cy3-Labeled) and Group with Addition of TPA (Cy5-Labeled) |
| Combination 3 | Negative Control (Cy3-Labeled) and Group with Addition of DGE and TPA (Cy5-Labeled) |
| Combination 4 | Group with Addition of TPA (Cy3-Labeled) and Group with Addition of DGE and TPA (Cy5-Labeled) |

(3) Hybridization

Ten microliters of a pre-hybridization solution (a solution containing 6 × SSC, 0.2% SDS, 5 × Denhardt's solution, 1 mg/mL denatured salmon sperm DNA solution passed through a filter having a size of 0.22 μm) was added

dropwise on a cover glass (22 ×22 mm), and covered with Human Cytokine CHIP (ver. 1.0) (manufactured by Takara Shuzo Co., Ltd., X104) with the side of the array DNA facing downward so that no gas bubbles entered onto the cover glass. Pre-hybridization was carried out by flipping the chip upside-down so that the side of the array DNA of the chip faced up, adding dropwise Paper Bond (manufactured by KOKUYO CO., LTD., Ta-100) to the periphery of the cover glass to seal the solution, and allowing the chip to stand at room temperature for 2 hours. The chip was transferred to 2 × SSC, and Paper Bond and the cover glass were removed, and the chip was washed with 2 × SSC and then with 0.2 × SSC, and thereafter dried by dissipating water by centrifugation.

The solution of fluorescence-labeled DNA dissolved in the above-mentioned hybridization solution was thermally denatured at 95°C for 2 minutes, and thereafter the solution was centrifuged to give supernatant. The supernatant was added dropwise on the cover glass (22 ×22 mm), covered with the chip treated as described above with the side of the array DNA of the chip facing downward so that no gas bubbles entered onto the cover glass. The chip was flipped upside-down so that the side of the array DNA of the chip faced up, and Paper Bond was added dropwise to the periphery of the cover glass, and the solution was sealed. This chip was placed in a wet box, and thereafter incubated in a thermostat at 65°C for 16 hours. The chip was transferred to 2 × SSC, and Paper Bond and the cover glass were removed, and the chip was washed in 2 × SSC, 0.2% SDS at 55°C for 30 minutes twice, thereafter washed in 2 × SSC, 0.2% SDS at 65°C for 5 minutes, and thereafter washed in 0.05 × SSC at room temperature for 5 minutes, and dried by dissipating water by centrifugation.

(4) Analysis

The fluorescence signal was scanned for each spot of the above-mentioned chip by using Affymetrix 418 Array Scanner (manufactured by Takara Shuzo Co., Ltd., GM105). The fluorescence signal was quantified for each spot by using ImaGene (manufactured by Takara Shuzo Co., Ltd., BD001) on the bases of the image data. As a result, the fluctuation in some of gene expressions could be confirmed in the group with addition of TPA, while the fluctuation can be confirmed to be made even larger in the group with addition of DGE + TPA. In other words, there could be confirmed that DGE has an action of modifying the fluctuation of gene by TPA in some of the genes. The results are summarized and shown in Table 5. Table 5 summarizes those genes of which fluctuations are modified by DGE among those genes showing fluctuation by TPA, wherein Group A-1 are those genes of which expressions are suppressed by DGE, and wherein Group B-1 are those genes of which expressions are accelerated by DGE.

Table 5

| Gene Name | GenBank Accession No. |
|---|---|
| Group A-1 | |
| Bone morphogenetic protein-6 (BMP-6) | NM_001718 |
| Chemokine receptor-1 (CCR-1) | NM_001295 |
| Hepatocyte growth factor (HGF) | X16323 |
| Intercellular adhesion molecule-1 (ICAM-1) | NM_000201 |
| Interleukin-1β (IL-1β) | X02532 |
| Interleukin-16 (IL-16) | M90391 |
| Leukemia inhibitory factor (LIF) | NM_002309 |
| Osteopontin | NM_000582 |
| Tumor necrosis factor-α (TNF-α) | X02910 |
| Neuropilin-1 | NM_003873 |
| Group B-1 | |
| Heme oxygenase-1 (HO-1) | NM_002133 |
| Thioredoxin reductase-1 | AF106697 |

Example 2

(1) Isolation and Storage of PBMCs

Four-hundred milliliters of blood was collected from a human normal individual donor. The collected blood was diluted 2-folds with PBS(-), overlaid on Ficoll-paque (manufactured by Pharmacia), and centrifuged at 500 × g for 20 minutes. The peripheral blood mononuclear cells (PBMCs) in the intermediate layer was collected with a pipette,

and washed with RPMI 1640 medium (manufactured by Bio Whittaker). The collected PBMCs were suspended in a storage solution of 90% FCS (manufactured by JRH Biosciences)/10% dimethyl sulfoxide, and stored in liquid nitrogen. During the experiment, these stored PBMCs were rapidly melted in water bath at 37°C, and washed with RPMI 1640 medium containing 10 μg/ml Dnase (manufactured by Calbiochem). Thereafter, the number of living cells was calculated by trypan blue staining method, and subjected to each experiment.

(2) Isolation of Plastic Adherent Monocytes

PBMCs were suspended in RPMI 1640 medium containing 5% human serum of blood type-AB, 0.1 mM nonessential amino acid, 1 mM sodium pyruvate, 2 mM L-glutamine (hereinabove manufactured by Bio Whittaker), 10 mM HEPES (manufactured by nakalai tesque), and 1% streptomycin-penicillin (manufactured by Gibco BRL) (hereinafter simply referred to as "5HRPMI medium") so as to have a concentration of $1 \times 10^7$ cells/mL. Thereafter, 15 ml of the suspension was spread on a 100-mm petri dish (manufactured by Asahi Techno Glass), and the cells were incubated in a wet-type incubator at 37°C in the presence of 5% $CO_2$ for 1.5 hours. Thereafter, the non-adherent cells were removed by suction, and each well was washed with RPMI 1640 medium, and 10 mL of the 5HRPMI medium was added to the wells, to give plastic adherent monocytes.

(3) Preparation of RNA

In each petri dish obtained in item (2) of Example 2, there was set a group in which 10 μL of an 1 μg/mL lipopolysaccharide (LPS, manufactured by Sigma, L-2012) aqueous solution thereto, and total RNA was collected 4 hours thereafter; or a group in which 10 μL of a 20 mM DGE aqueous solution was added thereto, and the cells were cultured for 4 hours, and thereafter, additional 10 μL of 1 μg/mL LPS aqueous solution was added thereto, and total RNA was collected 4 hours thereafter. Also, as the negative control, there was set a group in which 10 μL of water was added to a petri dish, and total RNA was collected 4 hours thereafter. Here, RNA was prepared by using RNeasy Mini Kit (manufactured by QIAGEN, 74104) in accordance with the instruction for the kit.

(4) Synthesis of cDNA

The synthesis of cDNA was carried out in accordance with the method described in item (2) of Example 1, provided that the combination of the solutions of fluorescence-labeled DNA was in accordance with that shown in Table 6.

Table 6

| | |
|---|---|
| Combination 1 | Negative Control (Cy3-Labeled) and Group with Addition of LPS (Cy5-Labeled) |
| Combination 2 | Negative Control (Cy3-Labeled) and Group with Addition of DGE and LPS (Cy5-Labeled) |

(5) Hybridization

Hybridization was carried out in accordance with the method described in item (3) of Example 1, provided that Human Cytokine CHIP (ver. 2.0) (manufactured by Takara Shuzo Co., Ltd., X104) was used as the chip.

(6) Analysis

The fluorescence signal was scanned for each spot of the above-mentioned chip by using Affymetrix 428 Array Scanner (manufactured by Takara Shuzo Co., Ltd.). The fluorescence signal was quantified for each spot by using ImaGene (manufactured by Takara Shuzo Co., Ltd., BD001) on the bases of the image data. As a result, in some of the genes, the increase in their expressions could be confirmed in the group with addition of LPS, while the increase could be confirmed to be suppressed by the addition of DGE. In addition in some of the genes, the fluctuation in their expressions could not be found in the group with addition of LPS, while the increase and the decrease in their expressions could be confirmed in the group with addition of DGE + LPS. The results are summarized and shown in Table 7. Table 7 summarizes those genes in which the increase in their expressions was suppressed by DGE among those genes showing the increase in their expressions by LPS (Group E); those genes of which expressions did not fluctuate by LPS, but were decreased by DGE + LPS (Group F); and those genes of which expressions were increased by DGE + LPS (Group G).

Table 7

| Gene Name | GenBank Accession No. |
|---|---|
| Group E | |
| Interleukin-2 receptor α (IL2RA) | X01057 |
| Interleukin-6 (IL6) | X04430 |
| CD166 | Y10183 |
| Interferon regulatory factor-1(IRF1) | X14454 |
| Interleukin-15 receptor α (IL15RA) | U31628 |
| Nuclear factor kappa-b p105 subunit (NFKB1) | M58603 |
| Caspase-1(CASP1) | M87507 |
| Interleukin -1β (IL1B) | M15330 |
| Tumor necrosis factor-α (TNF-α) | X02910 |
| Gro1 oncogene (GRO1) | X54489 |
| Interleukin -1α (IL1A) | M28983 |
| Inhibin βA (INHBA) | J03634 |
| Interleukin-7 receptor (IL7R) | M29696 |
| Precursor B cell colony enhancing factor (PBEF) | U02020 |
| Liver and activation regulated chemokine (LARC) | U64197 |
| Interleukin-8 (IL8) | M26383 |
| Group F | |
| Colony-stimulating factor-1 receptor (CSF1R) | X03663 |
| Chemokine (C-X-C motif) receptor-4 (CXCR4) | AF147204 |
| Endoglin (ENG) | NM_000118 |
| Group G | |
| Heme oxygenase-1 (HMOX1) | Z82244 |

Example 3 Preparation of DNA Microbeads and Screening of Genes

DNA microbeads were prepared as shown below in accordance with the method described in *Proc. Natl. Acad. Sci. USA,* **97,** 1665-1670 (2000) (hereinafter referred to as "Literature 1").

(1) Synthesis of cDNA

Human promyelocytic leukemia cell line HL-60 cells (ATCC CCL-240) were suspended in RPMI 1640 medium (manufactured by Bio Whittaker) containing 10% fetal bovine serum (manufactured by JRH Biosciences) and 1.3% dimethyl sulfoxide, and the cells were cultured at 37°C for one week in the presence of 5% carbon dioxide gas to induce differentiation to neutrophile-like cells. The cells obtained as described above were collected by centrifugation, and suspended in 10% fetal bovine serum-containing RPMI 1640 medium so as to have a concentration of $1 \times 10^6$ cells/ml. This suspension was added to two 10-cm petri dishes in an amount of 10 ml each. In one petri dish, 100 µl of water was added to the suspension and named (A); and in the other petri dish, 10 µl of a 100 µg/ml dimethyl sulfoxide solution of phorbol myristate acetate (TPA, manufactured by Gibco) was added to the suspension and named (B), and each of the cells was cultured for 4 hours. A whole RNA was prepared from each of the cells of (A) and (B) collected from each petri dish by using Trizol Reagent (manufactured by Gibco), and thereafter mRNA was purified from each whole RNA by using Oligotex™-dT30 (manufactured by Takara Shuzo Co., Ltd.). cDNA was synthesized by using cDNA synthesizing kit (manufactured by STRATAGENE) with 2.5 µg of the mRNA as a template. During the synthesis, as a primer used for reverse transcription, a 5'-biotin-labeled primer of the nucleotide sequence shown in SEQ ID NO: 1 of Sequence Listing was used.

The cDNA thus obtained was subjected to phenol-chloroform treatment, and purified by gel filtration using Sepharose CL4B (manufactured by Amersham-Pharmacia Biotech), and thereafter subjected to ethanol precipitation. cDNA collected as the precipitates was dissolved in 200 µl of *Dpn*II buffer (manufactured by New England Biolabs), and digestion was carried out with a restriction enzyme *Dpn*II (manufactured by New England Biolabs). A 3'-side portion of cDNA was collected from the resulting digest by using Streptoavidin Magnet Beads (manufactured by Dynal). There-

after, 20 μl of 10 × NEB #2 buffer (manufactured by New England Biolabs) was added thereto to adjust its total volume to 200 μl, and digestion was carried out with a restriction enzyme *Bsm*BI (manufactured by New England Biolabs). The reaction solution was subjected to phenol-chloroform treatment and ethanol precipitation. The resulting precipitates were dissolved in 10.5 μl of sterilized water, and the solution was used in the following experiment as a 3'-side cDNA solution.

(2) Hybridization of 3'-Side cDNA with Anti-Tagged Microbeads

Each of 1 μl of the 3'-side cDNA solutions derived from (A) and (B) synthesized in item (1) of Example 3 was inserted in 200 ng of a tag vector pLCV2 described in Literature 1, to give 6 plasmid pools, each pool comprising 140000 to 180000 clones. PCR was carried out for 48 runs for each plasmid pool using a 5'-biotin-labeled primer of the nucleotide sequence shown in SEQ ID NO: 2 of Sequence Listing and a 5'-FAM-labeled primer of the nucleotide sequence shown in SEQ ID NO: 3 of Sequence Listing, wherein each primer was used in an amount of 100 pmol per 100 μl of one reaction solution, and wherein 125 ng of each of the above-mentioned plasmid pool per one reaction solution was used as a template. PCR was carried out under the following conditions of:

keeping at 94°C for 3 minutes,
carrying out 8 cycles of reaction, wherein one cycle comprises 94°C for 30 seconds and 67°C for 3 minutes,
carrying out 22 cycles of reaction, wherein one cycle comprises 94°C for 30 seconds and 64°C for 3 minutes, and
keeping at 67°C for 3 minutes.

After the termination of the reaction, the PCR reaction solutions were collected together for each pool, and each reaction solution was subjected to phenol-chloroform treatment and ethanol precipitation. Thereafter, the resulting precipitates were dissolved in 1200 μl of sterilized water. One-hundred and eighty microliters of 10 × NEB #4 buffer (manufactured by New England Biolabs) was added thereto to adjust its total volume to 1800 μl, and digestion was carried out with a restriction enzyme *Pac*I (manufactured by New England Biolabs). A cDNA solution obtained by removing a fraction adsorbed to an Ultralink SA Resin (manufactured by Pierce) from the reaction solution was subjected to phenol-chloroform treatment and ethanol precipitation. Thereafter, the resulting precipitates were dissolved in 1200 μl of sterilized water. One-hundred and eighty microliters of 10 × NEB #2 buffer (manufactured by New England Biolabs) was added thereto to adjust its total volume to 1800 μl, and the solution was treated with T4 DNA Polymerase (manufactured by Takara Shuzo Co., Ltd.). During the treatment, only dGTP was added as a substrate to form a single-strand in the tagged portion.

Three-hundred micrograms of cDNA having this single-stranded tag for each pool was mixed with 144000000 anti-tagged microbeads described in Literature 1, and hybridization was carried out for 3 days in 400 μl of hybridization buffer (10 mM phosphate buffer (pH 7.2), 0.5 M NaCl, 0.01% Tween 20, 1.2% dextran sulfate), with shaking at 72°C. The microbeads were collected by centrifugation, and suspended in 500 μl of 10 mM Tris-HCl (pH 7.9), 10 mM $MgCl_2$, 50 mM NaCl, 1 mM dithiothreitol, 0.01% Tween 20, and the beads were washed at 64° to 70°C for 30 minutes, with shaking. After repeating this washing procedure several times, washed microbeads attached with cDNA for 6 pools were collected, and the fluorescence intensity (excitation wavelength: 488 nm, detection wavelength: 530 nm) of FAM for the microbeads was determined by using MoFlo Cytometer (manufactured by Dako Cytomation). One percent of the microbeads were collected from those having larger fluorescence intensity. By the above procedures, about 6000000 microbeads to which cDNA was bound via hybridization were obtained.

(3) Preparation of DNA Microbeads Suspension

About 2000000 microbeads among the microbeads to which cDNA was bound via hybridization, obtained in item (2) of Example 3 were suspended in 100 μl of 10 mM Tris-HCl (pH 7.9), 10 mM $MgCl_2$, 50 mM NaCl, 1.4 mM dithiothreitol, 0.01% Tween 20, 0.1 mM each of dATP, dGTP, dTTP, 5-methyl dCTP, 1 mM ATP, and 20 U T4 DNA Polymerase and 400 U T4 DNA Ligase were added thereto, and the mixture was reacted at 12°C for 2 hours. The reaction was stopped by pronase treatment, and thereafter the microbeads were collected by centrifugation from the reaction solution. The microbeads were suspended in 100 μl of *Dpn*II buffer (manufactured by New England Biolabs), and digestion was carried out with *Dpn*II (manufactured by New England Biolabs). The microbeads were collected by centrifugation, and suspended in 100 μl of 10 mM Tris-HCl (pH 7.5), 5 mM $MgCl_2$, 0.033 mM dGTP, 7.5 mM dithiothreitol, 0.01% Tween 20, and 10 U DNA Polymerase I Large Fragment (manufactured by Takara Shuzo Co., Ltd.) was added thereto, and the mixture was reacted at 25°C for 15 minutes. The microbeads were collected by centrifugation, and suspended in 100 μl of ligation buffer (manufactured by Takara Shuzo Co., Ltd.). Thereafter, 0.1 nmol of an adaptor previously prepared by annealing DNA of the nucleotide sequence shown in SEQ ID NO: 4 and SEQ ID NO: 5 of Sequence Listing was added thereto, and a ligation reaction was carried out by using T4 DNA Ligase (manufactured

by Takara Shuzo Co., Ltd.). The microbeads were collected by centrifugation, and suspended in beads stripping solution (150 mM NaOH, 0.01% Tween 20), and the reaction was carried out at room temperature for 10 minutes. The supernatant was removed from the reaction solution by centrifugation, and thereafter the microbeads were washed with 10 mM Tris-HCl (pH 8.0), 1 mM EDTA, 0.01% Tween 20, and the washed microbeads were suspended in 1 ml of 10 mM Tris-HCl (pH 8.0), 1 mM EDTA, 0.01% Tween 20, to give DNA microbeads suspensions derived from Sample 1 and Sample 2.

(4) Preparation of Probe

Each of 1 µl of the 3'-side cDNAs derived from (A) and (B) prepared in item (1) of Example 3 was inserted to 200 ng of probe vector pLCV described in Literature 1, to give plasmids of 8000000 to 10000000 clones. Twenty microliters of universal buffer H (manufactured by Takara Shuzo Co., Ltd.) was added to each of 10 µg of this plasmid to make up a total volume of 200 µl, and thereafter digestion was carried out with a restriction enzyme *Sau*3AI (manufactured by Takara Shuzo Co., Ltd.). Each of linear PCRs was carried out by using 2 nmol of 5'-FAM-labeled primer of the nucleotide sequence shown in SEQ ID NO: 6 of Sequence Listing with 1.2 µg of the *Sau*3AI-digested plasmid derived from (A) as a template; or using 4 nmol of 5'-Cy5-labeled primer of the nucleotide sequence shown in SEQ ID NO: 7 of Sequence Listing with 2.4 µg of the *Sau*3AI-digested plasmid derived from (B) as a template. The linear PCR was carried out under the following conditions of:

keeping at 95°C for 5 minutes carrying out 25 cycles of reaction, wherein one cycle comprises 95°C for 30 seconds, 64°C for 30 seconds, and 72°C for 30 seconds, and
keeping at 72°C for 10 minutes.

An amplified DNA was purified from each of the reaction mixture after the linear PCR by using QIAquick PCR Purification Kit (manufactured by QIAGEN), and the purified product was subjected to ethanol precipitation. Thereafter, the precipitates were dissolved in 21 µl of sterilized water, to give an FAM fluorescence-labeled probe derived from Sample 1 and a Cy5 fluorescence-labeled probe derived from Sample 2.

(5) Hybridization with DNA Microbeads

About 360000 each of the DNA microbeads derived from Sample 1 and the DNA microbeads derived from Sample 2 prepared in item (3) of Example 3 were mixed with each other. Five micrograms each of the FAM fluorescence-labeled probe derived from Sample 1 and the Cy5 fluorescence-labeled probe derived from Sample 2 prepared in item (4) were added to the beads. Hybridization was carried out overnight at 65°C in 50 µl buffer (4 × SSC, 25% formamide, 0.1% SDS) with shaking. The microbeads collected by centrifugation were washed in 1 × SSC, 0.1% SDS solution at 65°C for 30 minutes, and thereafter washed in 0.1 × SSC, 0.1% SDS solution at 65°C for 30 minutes. The washed DNA microbeads were collected by centrifugation, and suspended in 10 mM Tris-HCl (pH 8.0), 1 mM EDTA, 0.01% Tween 20 to give hybridized DNA microbeads.

(6) Sorting

The hybridized DNA microbeads obtained in item (5) of Example 3 were subjected to sorting by using MoFlo Cytometer (manufactured by Cytomation). The fluorescence intensity of FAM (excitation wavelength: 488 nm, detection wavelength: 530 nm) and the fluorescence intensity of Cy5 (excitation wavelength: 647 nm, detection wavelength: 670 nm) for the DNA microbeads were determined, and 3692 DNA microbeads having potent fluorescence intensity of FAM (beads suspension A) and 3349 DNA microbeads having potent fluorescence intensity of Cy5 (beads suspension B) were collected.

(7) PCR and DNA Nucleotide Sequencing

PCR was carried out by using each of primers of the nucleotide sequences shown in SEQ ID NO: 8 and SEQ ID NO: 9 of Sequence Listing with the beads suspension A or B collected in item (6) of Example 3 as a template. Two-hundred template beads and 40 pmol each of the primers were added to 100 µl of the reaction solution, and 5 runs of reactions were carried out for each of the beads suspension A and B. PCR was carried out under the following conditions of:

keeping at 95°C for 5 minutes,
carrying out 25 cycles of reaction, wherein one cycle comprises 95°C for 30 seconds, 64°C for 30 seconds, and

72°C for 30 seconds, and
keeping at 72°C for 10 minutes.

The reaction solutions of PCR were collected together for each of the suspensions A and B, and ligation was carried out by mixing 1 μl of each suspension with cloning vector pT7Blue (manufactured by Novagen), and thereby cloning was carried out. A plasmid was purified from 960 colonies derived from the beads suspension A and 960 colonies derived from the beads suspension B, a total of 1920 colonies, and nucleotide sequencing was carried out for an inserted DNA fragment. Among the nucleotide sequences obtained, those satisfying either one of the following conditions were selected.
Condition 1: The length of the insertion sequence being 40 bases or more.
Condition 2: The length of the insertion sequence being 30 bases or more and 40 bases or less, and not having polyA sequence.
When the same insertion sequence appears in plural plasmid, any one of the plasmids is selected. Finally, 740 plasmids were obtained as a template plasmid for DNA microarray.

Example 4 Analysis of Expression Using DNA Microarray

(1) Preparation of DNA Microarray

The reaction for PCR was carried out by using a primer pair having the sequences shown in SEQ ID NO: 10 and SEQ ID NO: 11 of Sequence Listing, with the 740 clones of plasmids obtained in Example 3 as a template. Next, the amplified product was purified by ethanol precipitation, and thereafter the product was dissolved in 1 × SolutionT (manufactured by Takara Shuzo Co., Ltd.) so as to have a concentration of 0.3 μg/μl, and this solution was used as a spotting solution. The spotting solution was spotted on MAS-coated slide glass (manufactured by Matsunami Glass), amino-group carrying slide glass, by using Affymetrix 417 Arrayer (manufactured by Affymetrix). The spotted glass was incubated at 37°C and humidity of 90% for 1 hour, and thereafter cross-linked by UV irradiation at energy of 60 $mJ/cm^2$. Further, the slide glass was washed once with 0.2% SDS and then twice with distilled water, and thereafter immersed in a blocking solution comprising 2.75 g of succinic anhydride, 162.5 ml of N-methyl-2-pyrrolidone and 15 ml of 1 M borate buffer (pH 8.0) for 20 minutes, to block free amino groups. Next, the slide glass was washed twice with distilled water and subjected to thermal denaturation in boiling water for 2 minutes, and rapidly cooled in 100% ethanol at ice temperature. Thereafter, the slide glass was dried by a low-speed centrifugation at 1000 rpm or so, and used for the following experiment.

(2) Preparation of Cells

HL-60 cells (ATCC CCL-240) were suspended in RPMI 1640 medium (manufactured by Bio Whittaker, 12-702F) containing 10% fetal bovine serum (manufactured by JRH Biosciences) and 1.3% dimethyl sulfoxide, and the cells were cultured at 37°C for one week in the presence of 5% carbon dioxide gas to induce differentiation to neutrophile-like cells. The cells obtained as described above were collected by centrifugation, and suspended in 10% fetal bovine serum-containing RPMI 1640 medium so as to have a concentration of $10^6$ cells/mL, and 10-mL portions of the cells were added to a 10-cm petri dish. In each petri dish, there was set a group in which 100 μL of a 4 mM DGE aqueous solution was added thereto, and RNA was collected 4 hours thereafter; a group in which 10 μL of a 100 μg/mL dimethyl sulfoxide solution of phorbol myristate acetate (TPA, manufactured by Gibco, 13139-019) was added thereto, and RNA was collected 4 hours thereafter; or a group in which 100 μL of a 4 mM DGE aqueous solution was added thereto, and the cells were cultured for 6 hours, and thereafter, additional 10 μL of 100 μg/mL TPA dimethyl sulfoxide solution was added thereto, and RNA was collected 4 hours thereafter. Also, as the negative control, there was set a group in which 100 μL of water was added to a petri dish, and RNA was collected 4 hours thereafter.

(3) Preparation of Labeled cDNA Used for Detection and Hybridization

A PolyA(+) RNA was prepared from HL-60 cells prepared in item (2) of Example 4 by using Trizol Reagent (manufactured by Gibco BRL) and Oligotex-dT30 <Super> (manufactured by Takara Shuzo Co., Ltd.) in accordance with the protocol for each of the kits. Next, each of cDNAs labeled with Cy3 and Cy5 was prepared by using RNA Fluorescence Labeling Core Kit (M-MLV Version) (manufactured by Takara Shuzo Co., Ltd.) in accordance with the protocol for the kit with 1.0 μg of PolyA(+) RNA as a template, and their cDNAs were mixed in the combinations of the sample RNA shown in Table 8.

Table 8

| Sample No. | Cy3 | Cy5 |
|---|---|---|
| 1 | Untreated | Treated with DGE |
| 2 | Untreated | Treated with TPA |
| 3 | Treated with TPA | Treated with DGE + Treated with TPA |
| 4 | Untreated | Treated with DGE + Treated with TPA |

Next, hybridization of the DNA microarray prepared in item (1) of Example 4 with the above-mentioned mixtures (4 kinds) of Cy3-labeled cDNA and Cy5-labeled cDNA was carried out in accordance with the instruction manual of IntelliGene (manufactured by Takara Shuzo Co., Ltd.), and the array was subjected to procedures of washing and drying. Subsequently, the fluorescence images were obtained for Cy3 (excitation wavelength: 532 nm, detection wavelength: 570 nm) and Cy5 (excitation wavelength: 635 nm, detection wavelength: 660 nm) by using Affymetrix 428 Array Scanner (manufactured by Affymetrix). Thereafter, an average of the signal intensities in the spot region of each spot, and an average and standard deviation of the signal intensities in the surrounding region of the spot (hereinafter referred to as "background region") were quantified and calculated by using an image quantification analyzing software ImaGene 4.1 (manufactured by Biodiscovery.

(4) Grouping of Each Clone

In order to group each clone according to statistical analysis on the basis of expression ratio, it is necessary to remove data showing low reliability, and data of clones not showing significant fluctuations. Accordingly, the clones subjected to grouping were selected by the following procedures. In other words, the threshold of the signal intensity was calculated from the average and standard deviation of the signal intensity in the background region obtained in item (3) of Example 4 in accordance with the following mathematical equation:

Threshold of Signal Intensity

= Average of Signal Intensity in Background Region +

2 × (Standard Deviation of Signal Intensity in Background Region)

The significance of the spot signal in each channel was judged by whether or not the average of the signal intensity of each spot region is greater than the above-mentioned threshold. Next, those clones of which spots do not have an effective signal in both Cy3 and Cy5 channels for all of the four samples were excluded. Further, the ratio of the signal intensity of Cy5 to that of Cy3 (hereinafter referred to as expression ratio) was calculated, and those clones of which expression ratios were greater than one-half and less than 2 for all of the samples were excluded in the later analysis as those clones not showing fluctuations. As a result of the above-mentioned selection, 227 clones were selected from 740 clones.

Next, the 227 clones selected as described above, of which expressions were fluctuated by the treatment with TPA and DGE, namely the clones having an expression ratio of Sample No. 3 or 4 of 0.55 or less or 1.8 or more, could be divided into the following four groups according to the relation of fluctuation by DGE treatment.

Group A-3: Clones of which expression is increased by the treatment with TPA and further decreased by the treatment with DGE

Group B-3: Clones of which expression is increased by the treatment with TPA and further increased by the treatment with DGE

Group C-3: Clones of which expression is decreased by the treatment with TPA and further decreased by the treatment with DGE

Group D-3: Clones of which expression is decreased by the treatment with TPA and further increased by the treatment with DGE

These clones were subjected to homology search. According to the results, the names of the nucleotide sequences acknowledged to be homologous with the clones were divided into the four groups, and their GenBank Accession Nos. are shown in Tables 9 and 10. In Tables 9 and 10, those clones of which homologies were acknowledged

are shown by the name of the nucleotide sequence as registered in GenBank, and those clones of which homologies were not acknowledged are shown by SEQ ID NO of Sequence Listing.

Table 9

| Nucleotide sequence of which homologies were Acknowledged | GenBank Accession No. |
|---|---|
| Group A-3 | |
| Homo sapiens signal transducer and activator of transcription 6 (STAT6) gene, exons 15 through 23 and complete cds | AF067575 |
| Homo sapiens cDNA: FLJ22298 fis, clone HRC04637 | AK025951 |
| Group B-3 | |
| Homo sapiens SUI1 isolog mRNA, complete cds | AF083441 |
| Homo sapiens hypothetical protein (HSPC014), mRNA | NM_015932 |
| Group C-3 | |
| Homo sapiens cDNA: FLJ22730 fis, clone HSI15793, highly similar to AF004162 Homo sapiens nickel-specific induction protein (Cap43) mRNA | AK026383 |
| Homo sapiens adenosine deaminase, RNA-specific (ADAR), transcript variant ADAR-c, mRNA | NM_015841 |
| Homo sapiens splicing factor 3b, subunit 2, 145 kD (SF3B2), mRNA | NM_006842 |
| Homo sapiens partial mRNA for double stranded RNA binding nuclear protein ILF3, alternative transcript | AJ271747 |
| Human transfer valyl-tRNA synthetase mRNA, 3' end of cds | M98326 |
| Nucleotide Sequence Shown in SEQ ID NO: 12 of Sequence Listing | None |

Table 10

| Nucleotide sequence of which homologies were Acknowledged | GenBank Accession No. |
|---|---|
| Group D-3 | |
| Homo sapiens cDNA: FLJ22997 fis, clone KAT11962, highly similar to HSEF1AC Human mRNA for elongation factor 1-alpha (clone CEF4) | AK026650 |
| Homo sapiens ribosomal protein L17 (RPL17) mRNA | NM_000985 |
| Homo sapiens ribosomal protein L24 (RPL24) mRNA | NM_000986 |
| Homo sapiens chromosome 17, clone hRPK.318_A_15, complete sequence | X75755 |
| Homo sapiens TPT1 gene for translationally controlled tumor protein (TCTP), exons 1-6 | AJ400717 |
| Homo sapiens ubiquinol-cytochrome c reductase binding protein (UQCRB) mRNA | NM_006294 |
| Homo sapiens ribosomal protein S15a (RPS15A) mRNA | NM_001019 |
| Homo sapiens ribosomal protein L27 (RPL27) mRNA | NM_000988 |

It was deduced from these results that each of the clones classified into Group A-3, Group B-3, Group C-3 and Group D-3 was a partial sequence of the genes shown in Table 11. As to those genes of which complete nucleotide sequence has not been elucidated, GenBank Accession Nos. of the nucleotide sequence shown in Tables 9 and 10 are given in GenBank Accession No. in Table 11.

Table 11

| Name of Genes | GenBank Accession No. |
|---|---|
| Group A-3 | |
| signal transducer and activator of transcription 6 (STAT6) | AF067575 |
| Gene Containing Homo Sapiens cDNA: FLJ22298 fis, clone HRC04637 | AK025951 |
| Group B-3 | |
| putative translation initiation factor (SUI1) | AF083441 |

Table 11 (continued)

| Name of Genes | GenBank Accession No. |
|---|---|
| Group B-3 | |
| Homo sapiens hypothetical protein (HSPC014) | NM_015932 |
| Group C-3 | |
| N-myc downstream regulated gene 1 (NDRG1) | NM_006096 |
| adenosine deaminase, RNA specific (ADAR) | NM_015841 |
| splicing factor 3b, subunit2 (SF3B2) | NM_006842 |
| interleukin enhancer binding factor 3 (ILF3) | NM_004516 |
| valyl-tRNA synthetase 2 (VARS2) | NM_006295 |
| Gene containing the nucleotide sequence shown in SEQ ID NO: 12 of Sequence Listing | None |
| Group D-3 | |
| eukaryotic translation elongation factor 1 alpha 1 (EEF1A1) | NM_001402 |
| ribosomal protein L17 (RPL17) | NM_000985 |
| ribosomal protein L24 (RPL24) | NM_000986 |
| splicing factor, arginine/serine-rich 2 (SFRS2) | NM_003016 |
| tumor protein, translationally-controlled 1 (TPT1) | NM_003295 |
| ubiquinol-cytochrome c reductase binding protein (UQCRB) | NM_006294 |
| ribosomal protein S15a (RPS15A) | NM_001019 |
| ribosomal protein L27 (RPL27) | NM_000988 |

## SEQUENCE FREE TEXT

The sequence of SEQ ID NO: 1 is a sequence for reverse transcription primer for total RNA.
The sequence of SEQ ID NO: 2 is a sequence for PCR primer for amplifying a portion of TagVector pLCV2.
The sequence of SEQ ID NO: 3 is a sequence for PCR primer for amplifying a portion of TagVector pLCV2.
The sequence of SEQ ID NO: 4 is a sequence for an oligonucleotide adaptor.
The sequence of SEQ ID NO: 5 is a sequence for an oligonucleotide adaptor.
The sequence of SEQ ID NO: 6 is a sequence for linear PCR primer for amplifying a portion of ProbeVector pLCV.
The sequence of SEQ ID NO: 7 is a sequence for linear PCR primer for amplifying a portion of ProbeVector pLCV.
The sequence of SEQ ID NO: 8 is a sequence for PCR primer for cDNA on DNA microbeads.
The sequence of SEQ ID NO: 9 is a sequence for PCR primer for cDNA on DNA microbeads.
The sequence of SEQ ID NO: 10 is a sequence for PCR primer for amplifying a portion of pT7Blue Vector.
The sequence of SEQ ID NO: 11 is a sequence for PCR primer for amplifying a portion of pT7Blue Vector.

## INDUSTRIAL APPLICABILITY

The present invention provides a method of screening a physiologically active substance or a candidate substance for a physiologically active substance, possessing gene controlling activity like that of DGE possessing various physiological activities; an agent for controlling gene effective for maintaining homeostasis in a living body, for instance, for mitigation or prevention of stress; and a method for controlling a gene. According to the present invention, there can be expected, for instance, therapeutic or prophylactic effects for diseases or symptoms on which the expression of various physiological activities owned by DGE effectively acts.

## Claims

1. A method of screening a biologically active substance, **characterized in that** the method comprises the steps of:

   (1) loading to a living body either 1) a test substance or 2) a compound of the following (i):

   (i) at least one compound selected from the group consisting of a compound represented by the following formula (I):

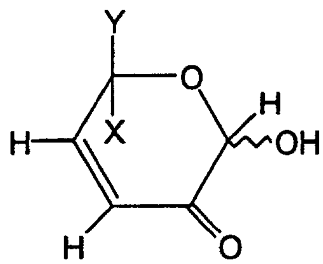

(I)

wherein X and Y are H or $CH_2OH$, with proviso that when X is $CH_2OH$, Y is H, and when X is H, Y is $CH_2OH$,
a derivative thereof and a salt thereof; and/or
a compound of the following (ii):

   (ii) at least one compound selected from the group consisting of 3,6-anhydrogalactose represented by the following formula (II):

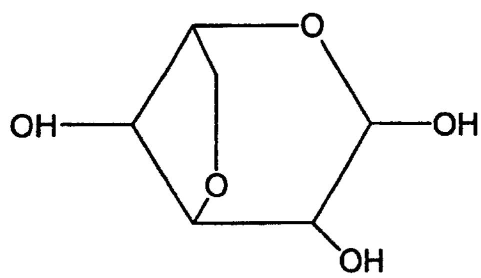

(II)

,

an aldehyde thereof, a hydrate thereof, and a 2-O-methylated product and a 2-O-sulfated product of the foregoing compounds; and/or at least one compound selected from the group consisting of soluble saccharide compounds having said compound at reducing end;

   (2) determining an expression level of a gene in the living body of said (1), and comparing resulting values between a case where 1) is loaded and a case where 2) is loaded; and
   (3) selecting a test substance which causes a change in an expression level of at least one gene to substantially the same extent as a change where 2) is loaded.

2. A method of screening a biologically active substance, **characterized in that** the method comprises the steps of:

   (1) loading a test substance to a living body;
   (2) determining an expression level of a gene in the living body of said (1) and an expression level of a gene in the living body without the loading, and comparing resulting values therebetween; and
   (3) selecting a test substance which decreases an expression level of at least one gene selected from the following Group A, Group C, Group E and Group F, and/or increases an expression level of at least one gene selected from the following Group B, Group D and Group G:

Group A:

Bone morphogenetic protein-6 [BMP-6 (GenBank Accession No. NM_001718)] gene,
Chemokine receptor-1 [CCR-1 (GenBank Accession No. NM_001295)] gene,
Hepatocyte growth factor [HGF (GenBank Accession No. X16323)] gene,
Intercellular adhesion molecule-1 [ICAM-1 (GenBank Accession No. NM_000201)] gene,
Interleukin-1β [IL-1β (GenBank Accession No. X02532)] gene,
Interleukin-16 [IL-16 (GenBank Accession No. M90391)] gene,
Leukemia inhibitory factor [LIF (GenBank Accession No. NM_002309)] gene,
Osteopontin [Osteopontin (GenBank Accession No. NM_000582)] gene,
Tumor necrosis factor-α [TNF-α (GenBank Accession No. X02910)] gene,
Neuropilin-1 [Neuropilin-1 (GenBank Accession No. NM_003873)] gene, signal transducer and activator of transcription 6 [STAT6 (GenBank Accession No. AF067575)] gene, and
Gene containing Homo Sapiens cDNA: FLJ22298 fis, clone HRC04637 (GenBank Accession No. AK025951),

Group B:

Heme oxygenase-1 [HO-1 (GenBank Accession No. NM_002133)] gene,
Thioredoxin reductase-1 (GenBank Accession No. AF106697) gene, putative translation initiation factor [SUI1 (GenBank Accession No. AF083441)] gene, and
Homo sapiens hypothetical protein [HSPC014 (GenBank Accession No. NM_015932)] gene;

Group C:

N-myc downstream regulated gene 1 [NDRG1 (GenBank Accession No. NM_006096)] gene,
adenosine deaminase, RNA specific [ADAR (GenBank Accession No. NM_015841)] gene,
splicing factor 3b, subunit2 [SF3B2 (GenBank Accession No. NM_006842)] gene,
interleukin enhancer binding factor 3 [ILF3 (GenBank Accession No. NM_004516)] gene,
valyl-tRNA synthetase 2 [VARS2 (GenBank Accession No. NM_006295)] gene, and
Gene containing the nucleotide sequence shown in SEQ ID NO: 12 of Sequence Listing;

Group D:

eukaryotic translation elongation factor 1 alpha 1 [EEF1A1 (GenBank Accession No. NM_001402)] gene,
ribosomal protein L17 [RPL17 (GenBank Accession No. NM_000985)] gene,
ribosomal protein L24 [RPL24 (GenBank Accession No. NM_000986)] gene,
splicing factor, arginine/serine-rich 2 [SFRS2 (GenBank Accession No. NM_003016)] gene,
tumor protein, translationally-controlled 1 [TPT1 (GenBank Accession No. NM_003295)] gene,
ubiquinol-cytochrome c reductase binding protein [UQCRB (GenBank Accession No. NM_006294)] gene,
ribosomal protein S15a [RPS15A (GenBank Accession No. NM_001019)] gene, and
ribosomal protein L27 [RPL27 (GenBank Accession No. NM_000988)] gene;

Group E:

Interleukin-2 receptor α [IL2RA (GenBank Accession No. X01057)] gene,
Interleukin-6 [IL6 (GenBank Accession No. X04430)] gene,
CD166 (GenBank Accession No. Y10183) gene,
Interferon regulatory factor-1 [IRF1 (GenBank Accession No. X14454)] gene,
Interleukin-15 receptor α [IL15RA (GenBank Accession No. U31628)] gene,
Nuclear factor kappa-b p105 Subunit [NFKB1 (GenBank Accession No. M58603)] gene,
Caspase-1 [CASP1 (GenBank Accession No. M87507)] gene,
Interleukin-1β [IL1B (GenBank Accession No. M15330)] gene,
Tumor necrosis factor-α [TNF-α (GenBank Accession No. X02910)] gene,
Gro1 oncogene [GRO1 (GenBank Accession No. X54489)] gene,
Interleukin-1α [IL1A (GenBank Accession No. M28983)] gene,

Inhibin βA [INHBA (GenBank Accession No. J03634)] gene,
Interleukin-7 receptor [IL7R (GenBank Accession No. M29696)] gene,
Precursor B cell colony enhancing factor [PBEF (GenBank Accession No. U02020)] gene,
Liver and activation regulated chemokine [LARC (GenBank Accession No. U64197)] gene, and
Interleukin-8 [IL8 (GenBank Accession No. M26383)] gene;

Group F:

Colony-stimulating factor-1 receptor [CSF1R (GenBank Accession No. X03663)] gene,
Chemokine (C-X-C motif) receptor-4 [CXCR4 (GenBank Accession No. AF147204)] gene, and
Endoglin [ENG (GenBank Accession No. NM_000118)] gene; and

Group G:

Heme oxygenase-1 [HMOX1 (GenBank Accession No. Z82244)] gene.

**3.** A method of screening a biologically active substance, **characterized in that** the method comprises the steps of:

(1) loading stress to a living body, and loading a test substance with stress to a living body, respectively;
(2) determining each of expression levels of a gene in the living body of said (1) and in a normal living body without any of the loading; and
(3) comparing the expression levels of a gene obtained in said (2) between the living body of said (1) and the normal living body, and selecting a test substance showing at least one gene expression pattern selected from the gene expression patterns of the followings (a) to (g):

(a) an expression level of at least one gene selected from Group A as defined in claim 2 increases when stress is loaded to a living body, and decreases when a test substance and stress are loaded to a living body, as compared to that of the normal living body;
(b) an expression level of at least one gene selected from Group B as defined in claim 2 increases when stress is loaded to a living body, and further increases when a test substance and stress are loaded to a living body, as compared to that of the normal living body;
(c) an expression level of at least one gene selected from Group C as defined in claim 2 decreases when stress is loaded to a living body, and further decreases when a test substance and stress are loaded to a living body, as compared to that of the normal living body;
(d) an expression level of at least one gene selected from Group D as defined in claim 2 decreases when stress is loaded to a living body, and increases when a test substance and stress are loaded to a living body, as compared to that of the normal living body;
(e) an expression level of at least one gene selected from Group E as defined in claim 2 increases when stress is loaded to a living body, and decreases when a test substance and stress are loaded to a living body, as compared to that of the normal living body;
(f) an expression level of at least one gene selected from Group F as defined in claim 2 is the same extent as that of the normal living body when stress is loaded to a living body, and decreases when a test substance and stress are loaded to a living body, as compared to that of the normal living body; and
(g) an expression level of at least one gene selected from Group G as defined in claim 2 is the same extent as that of the normal living body when stress is loaded to a living body, and increases when a test substance and stress are loaded to a living body, as compared to that of the normal living body.

**4.** The method according to claim 3, wherein the stress is a treatment with phorbol myristate acetate, and wherein the gene expression pattern is selected from the gene expression patterns of said (a) to (d).

**5.** The method according to claim 3, wherein the stress is a treatment with lipopolysaccharide, and wherein the gene expression pattern is selected from the gene expression patterns of said (e) to (g).

**6.** The method according to any one of claims 1 to 5, wherein the living body is an organism individual or a cultured cell.

**7.** The method according to any one of claims 1 to 6, wherein the expression level of a gene is determined by an amount of mRNA transcribed from the gene.

**8.** The method according to claim 7, wherein the expression level of a gene is determined by hybridization method using a DNA array.

**9.** A DNA array usable for the method of claim 8, **characterized in that** at least two genes selected from the genes of Groups A to G as defined in claim 2 or fragments thereof are immobilized on a support at a given position.

**10.** A gene controlling agent **characterized in that** the gene controlling agent comprises a compound possessing an action of decreasing an expression level of at least one gene selected from Group A, Group C, Group E and Group F as defined in claim 2, and/or increasing an expression level of at least one gene selected from Group B, Group D and Group G as defined in claim 2.

**11.** A gene controlling agent **characterized in that** the gene controlling agent comprises a compound possessing an action of decreasing an expression level of at least one gene selected from Group A, Group C, Group E and Group F as defined in claim 2, and/or increasing an expression level of at least one gene selected from Group B, Group D and Group G as defined in claim 2, in a living body to which stress is loaded.

**12.** A gene controlling agent **characterized in that** the gene controlling agent comprises a compound possessing an action of decreasing an expression level of at least one gene selected from Group A and Group E as defined in claim 2 in a living body to which stress is loaded, wherein the expression level of at least one gene is increased by the stress.

**13.** A gene controlling agent **characterized in that** the gene controlling agent comprises a compound possessing an action of increasing an expression level of at least one gene selected from Group B and Group G as defined in claim 2 in a living body to which stress is loaded, wherein the expression level of at least one gene is increased by the stress.

**14.** A gene controlling agent **characterized in that** the gene controlling agent comprises a compound possessing an action of decreasing an expression level of at least one gene selected from Group C and Group F as defined in claim 2 in a living body to which stress is loaded, wherein the expression level of at least one gene is decreased by the stress.

**15.** A gene controlling agent **characterized in that** the gene controlling agent comprises a compound possessing an action of increasing an expression level of at least one gene selected from Group D as defined in claim 2 in a living body to which stress is loaded, wherein the expression level of at least one gene is decreased by the stress.

**16.** The gene controlling agent according to any one of claims 10 to 15, wherein the compound is a substance obtained by the method of any one of claims 1 to 8.

**17.** The gene controlling agent according to any one of claims 10 to 16, wherein the gene control agent is purposed for mitigating or preventing stress.

**18.** A method of controlling a gene **characterized by** administering to a living body either at least one compound selected from the group consisting of compounds of (i) and (ii) as defined in claim 1 and a compound possessing an activity for controlling gene expression to substantially the same extent as that of said compounds for at least one gene, or a composition comprising the compound, thereby decreasing an expression level of at least one gene selected from Group A, Group C, Group E and Group F, and/or increasing an expression level of at least one gene selected from Group B, Group D and Group G, wherein Groups A to G are as defined in claim 2.

**19.** A method of controlling a gene **characterized by** administering to a living body to which stress is loaded either at least one compound selected from the group consisting of compounds of (i) and (ii) as defined in claim 1 and a compound possessing an activity for controlling gene expression to substantially the same extent as that of said compounds for at least one gene, or a composition comprising the compound, thereby decreasing an expression level of at least one gene selected from Group A, Group C, Group E and Group F, and/or increasing an expression level of at least one gene selected from Group B, Group D and Group G, wherein Groups A to G are as defined in claim 2.

**20.** The method according to claim 18 or 19, wherein the compound possessing an activity for controlling gene expression to substantially the same extent as that of compounds of (i) and (ii) in claim 1 for at least one gene is a

substance obtained by the method of any one of claims 1 to 8.

**21.** A substance obtainable by the method of any one of claims 1 to 8.

**22.** The substance according to claim 21, which is a substance derived from a plant, a substance derived from Basidiomycetes, or a derivative thereof.

**23.** The substance according to claim 21 or 22, which is a polysaccharide, a degradation product thereof, or a derivative thereof.

## INTERNATIONAL SEARCH REPORT

International application No.
PCT/JP01/08698

A. CLASSIFICATION OF SUBJECT MATTER
Int.Cl[7] C12Q 1/68, C12N 15/09, C12M 1/00, G01N 33/15, G01N 33/50, G01N 33/53, G01N 33/566, A61K 31/351, A61K 45/00

According to International Patent Classification (IPC) or to both national classification and IPC

B. FIELDS SEARCHED

Minimum documentation searched (classification system followed by classification symbols)
Int.Cl[7] C12Q 1/68, C12N 15/09, C12M 1/00, G01N 33/15, G01N 33/50, G01N 33/53, G01N 33/566, A61K 31/351, A61K 45/00

Documentation searched other than minimum documentation to the extent that such documents are included in the fields searched

Electronic data base consulted during the international search (name of data base and, where practicable, search terms used)
REGISTRY (STN), CA (STN), MEDLINE (STN), JICST FILE (JOIS)

C. DOCUMENTS CONSIDERED TO BE RELEVANT

| Category* | Citation of document, with indication, where appropriate, of the relevant passages | Relevant to claim No. |
|---|---|---|
| X | Ryuuji ENOKI et al. "Kanten Yurai Agaro-Origo-tou ni yoru Heme Oxygenase-1 Yuudou to Kakushu Seiri Kassei tono Soukan oyobi DNA Chip wo mochiita Idenshi Hatsugen no Kaiseki", Dai 59kai Nippon Gan Gakkai Soukai Kiji, 01 September, 2000, page 215 | 1-17,21-23 |
| P,X | WO 01/51480 A1 (Takara Shuzo Co., Ltd.), 19 July, 2001 (19.07.01) (Family: none) | 1-17,21-23 |
| X | WO 00/43018 A1 (Takara Shuzo Co., Ltd.), 27 July, 2000 (27.07.00) (Family: none) | 1-17,21-23 |
| X | WO 00/43407 A1 (Takara Shuzo Co., Ltd.), 27 July, 2000 (27.07.00), & EP 1146050 A1 | 1-17,21-23 |
| X | WO 99/64424 A1 (Takara Shuzo Co., Ltd.), 16 December, 1999 (16.12.99), & AU 9940606 A & EP 1086952 A1 | 1-17,21-23 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 January, 2002 (07.01.02) | 22 January, 2002 (22.01.02) |
| Name and mailing address of the ISA/ Japanese Patent Office | Authorized officer |
| Facsimile No. | Telephone No: |

Form PCT/ISA/210 (second sheet) (July 1992)

**INTERNATIONAL SEARCH REPORT**

International application No.
PCT/JP01/08698

C (Continuation). DOCUMENTS CONSIDERED TO BE RELEVANT

| Category* | Citation of document, with indication, where appropriate, of the relevant passages | Relevant to claim No. |
|---|---|---|
| A | WO 99/24447 A1 (Takara Shuzo Co., Ltd.), 20 May, 1999 (20.05.99), & AU 9910517 A & EP 1038879 A1 | 1-17,21-23 |
| A | JP 6-080565 A (Ishihara Sangyo Kaisha, Ltd.), 22 March, 1994 (22.03.94) (Family: none) | 1-17,21-23 |

Form PCT/ISA/210 (continuation of second sheet) (July 1992)

**INTERNATIONAL SEARCH REPORT**

International application No.
PCT/JP01/08698

**Box I Observations where certain claims were found unsearchable (Continuation of item 1 of first sheet)**

This international search report has not been established in respect of certain claims under Article 17(2)(a) for the following reasons:

1. ☒ Claims Nos.: 18-20
because they relate to subject matter not required to be searched by this Authority, namely:

Claims 18 to 20 substantially pertain to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The invention as set forth in claim 1 is an invention relating to a method of screening a physiologically active substance by loading a specific compound on a living body and quantifying the expression dose of a gene in the living body. In contrast, the invention as set forth in claim 2 is an invention relating to a method of screening a physiologically active substance by loading test substances on living bodies and thus selecting a test substance causing a decrease in the expression dose of a gene selected among specific genes and/or a test substance causing an increase in the expression dose of a gene selected among specific genes. Therefore, these inventions are not considered as forming a single general inventive concept. Such being the case, the inventions as set forth in claims 1 to 17 and claims 21 to 23 are divided into two groups of inventions which are not considered as relating to a group of inventions so linked as to form a single general inventive concept.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☒ *No protest accompanied the payment of additional search fees.*

Form PCT/ISA/210 (continuation of first sheet (1)) (July 1992)

# SEQUENCE LISTING

<110> Takara Shuzo Co., Ltd.

<120> A screening method for biologically active substance

<130> 01-065-PCT

<150> JP 2000-303644
<151> 2000-10-03

<150> JP 2001-283393
<151> 2001-09-18

<160> 12

<210> 1
<211> 43
<212> DNA
<213> Artificial Sequence

<220>
<223> Reverse transcription primer for total mRNA

<400> 1
gacatgctgc attgagacga ttcttttt ttttttttt ttv 43

<210> 2

<211> 25

<212> DNA

<213> Artificial Sequence

<220>

<223> PCR primer to amplify a portion of TagVector pLCV2

<400> 2

cgccagggtt ttcccagtca cgacg 25

<210> 3

<211> 24

<212> DNA

<213> Artificial Sequence

<220>

<223> PCR primer to amplify a portion of TagVector pLCV2

<400> 3

gcttccggct cgtatgttgt gtgg 24

<210> 4

<211> 14

<212> DNA

<213> Artificial Sequence

<220>

<223> Designed oligonucleotide adapter component

<400> 4

gactggcagc tcgt 14

<210> 5

<211> 17

<212> DNA

<213> Artificial Sequence

<220>

<223> Designed oligonucleotide adapter component

<400> 5

atcacgagct gccagtc 17

<210> 6

<211> 18

<212> DNA

<213> Artificial Sequence

<220>

<223> Linear PCR primer to amplify a portion of ProbeVector pLCV

<400> 6

tgtaaaacga cggccagt 18

<210> 7

<211> 18

<212> DNA

<213> Artificial Sequence

<220>

<223> Linear PCR primer to amplify a portion of ProbeVector pLCV

<400> 7

tgtaaaacga cggccagt 18

<210> 8

<211> 19

<212> DNA

<213> Artificial Sequence

<220>

<223> PCR primer for cDNA inserted at DNA microbeads

<400> 8

gcccgcattg agacgattc 19

<210> 9

<211> 18

<212> DNA

<213> Artificial Sequence

<220>

<223> PCR primer for cDNA inserted at DNA microbeads

<400> 9

gattggcagc tcgtgatc 18

<210> 10

<211> 20

<212> DNA

<213> Artificial Sequence

<220>

<223> PCR primer to amplify a portion of pT7Blue vector

<400> 10

agggttttcc cagtcacgac 20

<210> 11

<211> 20

<212> DNA

<213> Artificial Sequence

<220>

<223> PCR primer to amplify a portion of pT7Blue vector

<400> 11

atgcttccgg ctcgtatgtt 20

<210> 12
<211> 73
<212> DNA
<213> Homo sapiens

<400> 12
ttttttttt ttttgtcatg ttggagttat gaggggaatg gggagttgtt tgtttccatt 60
tgtaaactcg atc 73